# EUROPEAN PATENT APPLICATION

(11) **EP 4 389 018 A1**
(43) Date of publication of application: **26.06.2024**
(21) Application number: 23220044.4
(22) Date of filing: 22.12.2023
(51) Int. Cl.: A61B 17/04

(54) **SOFT TISSUE SECUREMENT DEVICE AND ASSOCIATED METHODS**

(30) Priority: 22.12.2022 US 202263434779 P
(71) Applicant: Stryker Corporation, Kalamazoo, MI 49002 (US)
(72) Inventor: CALLISON, Ross, Denver, 80237 (US); BIXBY, Elliot Ford, Milwaukie, 97267 (US); COOK, Nathan, Portland, 97213 (US); PILGERAM, Kyle Craig, San Jose, 95123 (US)
(74) Representative: V.O.

(57) **Abstract**

A device for securing soft tissue to bone includes a suture anchor, a first suture portion, a second suture portion and a working suture. The suture anchor has a lumen therethrough. The first suture portion has a length extending from a first end to a second end and passes into and out of the lumen over at least part of the length moving toward the second end from the first end. The second suture portion has a length extending from a first end to a second end and is disposed relative to the suture anchor such that at least part of the second suture portion encircles the suture anchor circumferentially around an outer surface of the suture anchor at least one complete rotation. The working suture has a first end operatively connected to at least one of the suture anchor, the first suture portion and the second suture portion.

## Description

### CROSS-REFERENCE TO RELATED APPLICATION

This application claims the benefit of the filing date of U.S. Provisional Patent Application No. 63/434,779, filed December 22, 2022, the disclosure of which is hereby incorporated by reference herein in its entirety.

### BACKGROUND OF THE INVENTION

Traditional suture anchors are typically made of metal or hard plastic and include a structure which connects or otherwise secures a filament, such as a suture, or a portion of tissue, to a body of the anchor. Such devices may be fabricated with a diameter suitable for disposal of the device in a bone, and may be anchored with wings, barbs, threads or the like. However, such traditional devices tend to be large in diameter, and must include sufficient material, or other additional structures, to withstand the forces pulling against the device. This means that a significant amount of bone mass is needed in the location receiving the anchor. As a result, the size of traditional suture anchors may limit the possible implantation locations in the body. One approach taken to address this issue has been to create "all-suture" anchors made solely from suture-like material.

While "all-suture" anchors may be more versatile than traditional suture anchors in that they may be disposed in smaller bone holes, such devices would still benefit from a minimization in the number of knots necessary to fully secure the anchor in the body, as well as better techniques to allow for their varied utilization during a surgical procedure. One way to minimize knots has been to use knotless fixation elements to affix structures together, such as soft tissue to a suture anchor. One example of a knotless fixation element is a so-called "Chinese finger trap" design whereby a length of suture passes through itself and/or another length of suture to create a high-friction engagement between the co-axial suture material, much like how the children's toy of the same name operates. However, current finger trap designs used with all-suture anchors may suffer from weak fixation (e.g., slippage within the finger trap), and complex use. This is particularly the case in designs where the finger trap is formed by an operator during surgery.

Thus, a need exists for suture anchor configurations that are easy to use and have the ability to withstand higher forces experienced within the repair.

### BRIEF SUMMARY OF THE INVENTION

In a first aspect, the present disclosure relates to a device for securing soft tissue to bone. The device includes a suture anchor, a first suture, a shuttle and a second suture. The suture anchor is capable of being positioned within a bone hole, the suture anchor including a first end, a second end, an outer wall extending along a length between the first end and the second end, and a hollow interior extending along at least a portion of the length. The first suture includes a first end portion, a second end portion, and a hollow braid, and passes through the outer wall of the suture anchor at least once. At least a portion of the first suture is positioned within at least a portion of the hollow interior of the suture anchor. The shuttle includes a suture-engaging structure, and is capable of being positioned within at least a portion of the hollow braid of the first suture. The second suture includes a first end portion and a second end portion. One of the first or second end portions of the second suture is coupled to the first suture, the other of the first or second end portions of the second suture being capable of being coupled to the suture engaging structure. The shuttle is capable of being actuated to move the other of the first or second end portions of the second suture into the hollow braid of the first suture.

Optionally, the shuttle is positioned within at least the portion of the hollow braid of the first suture, the portion of the hollow braid of the first suture is positioned outside of the hollow interior of the suture anchor. Optionally the first suture is a cylindrical length of suture and/or the second suture is a, e.g. substantially, flat length of suture. Optionally, one of the first or second end portions of the second suture is coupled to one of the first or second end portions of the first suture by a chain-link structure. Optionally, one of the first or second end portions of the second suture is coupled to one of the first or second end portions of the first suture by an opposing end-to-end tuck splice. Optionally, the device may include first and second sutures such that one of the first or second end portions of the second suture is coupled to one of the first or second end portions of the first suture by opposing finger trap structures. Optionally, at least two portions of the first suture may be positioned outside of the hollow interior of the suture anchor, and the shuttle may be positioned within the at least two portions of the first suture positioned outside of the hollow interior of the suture anchor. The shuttle may be capable of being actuated to move the other of the first or second end portions of the second suture into the hollow braid of the at least two portions of the first suture positioned outside of the hollow interior of the suture anchor. Optionally, none of the second suture is positioned within the hollow interior of the suture anchor. Optionally, one of the first or second end portions of the second suture is coupled to the first suture without passing through the hollow braid of the first suture. Optionally, the first suture includes one or more splices through itself. Optionally, the portion of the first suture is positioned within at least the portion of the hollow interior of the suture anchor and another portion of the first suture encircles the suture anchor circumferentially around the outer wall at least one half of a complete rotation, such as at least one complete rotation. The shuttle is capable of being positioned within the hollow braid of the portion of the first suture encircling the suture anchor.

Optionally, the device may be arranged so that when the shuttle is positioned within at least the portion of the hollow braid of the first suture, the portion of the hollow braid of the first suture is positioned outside of the hollow interior of the suture anchor. Optionally, the device may be arranged so that the one of the first or second end portions of the second suture is coupled to the first suture without passing through the hollow braid of the first suture. Optionally, the device may be arranged so that the first suture includes one or more splices through itself.

In a second aspect, a device includes a suture anchor, a first suture, a shuttle and a second suture. The suture anchor is capable of being positioned within a bone hole and includes a first end, a second end, an outer wall extending along a length between the first end and the second end, and a hollow interior extending along at least a portion of the length. The first suture includes a first end portion, a second end portion, and a hollow braid. The first suture passes through the outer wall of the suture anchor at least once. A portion of the first suture is positioned within at least a portion of the hollow interior of the suture anchor and another portion of the first suture encircles the suture anchor circumferentially around the outer wall at least one half of a complete rotation, such as at least one complete rotation. The shuttle includes a suture-engaging structure and is capable of being positioned within the hollow braid of the portion of the first suture encircling the suture anchor. The second suture includes a first end portion and a second end portion. One of the first or second end portions of the second suture is coupled to one of the first or second end portions of the first suture, while the other of the first or second end portions of the second suture is capable of being coupled to the suture-engaging structure. Additionally, the shuttle is capable of being actuated to move the other of the first or second end portions of the second suture into the hollow braid of the first suture.

Optionally, the first suture may include a third end portion and first and second portions. Additionally, the device may be arranged such that the second end portion of the first suture is coupled to one of the first or second end portions of the second suture. Further, the first portion of the first suture may extend to the first end portion and the second portion of the first suture may extend to the third end portion.

Optionally, the first portion of the first suture is the portion of the first suture encircling the suture anchor circumferentially around the outer wall at least one complete rotation. Optionally, the second portion of the first suture passes through the outer wall of the suture anchor at least twice. Optionally, the first end portion and the third end portion of the first suture are coupled together and are positioned within the hollow interior of the suture anchor, through one of the first end or second end of the suture anchor from within the hollow interior to extending beyond one of the first end or second end of the suture anchor. Optionally, the second end portion of the first suture includes a loop and the first portion and second portion of the first suture each extend from the loop. Optionally, one of the first or second end portions of the second suture includes a loop, the loop of the first suture coupled to the loop of the second suture to form a chain-link. Optionally, the first portion of the first suture passes in between the second portion of the first suture and the outer wall of the suture anchor at least once.

Optionally, the device is arranged such that at least two portions of the second portion of the first suture are positioned outside of the hollow interior of the suture anchor, and a second shuttle including a length and a second suture-engaging structure is positioned within the at least two portions of the second portion of the first suture positioned outside of the hollow interior of the suture anchor. In this arrangement, the second shuttle, capable of being coupled to the suture-engaging structure, is capable of being actuated to move the other of the first or second end portions of the second suture into the hollow braid of the at least two portions of the second portion of the first suture positioned outside of the hollow interior of the suture anchor. Optionally, the first suture is a substantially cylindrical length of suture and the second suture is a substantially flat length of suture. Optionally, one of the first or second end portion of the second suture is coupled to one of the first or second end portions of the first suture by an opposing end-to-end tuck splice. Optionally, one of the first or second end portions of the second suture is coupled to one of the first or second end portions of the first suture by opposing finger trap structures.

In a third aspect, a device includes a suture anchor, a first suture portion, a second suture portion and a working suture. The suture anchor has a lumen coaxial with a length of the suture anchor. The first suture portion has a length extending from a first end to a second end and passes into and out of the lumen over at least part of the length moving toward the second end from the first end. The second suture portion has a length extending from a first end to a second end and is disposed relative to the suture anchor such that a position of the first suture portion keeps the second suture portion proximate the suture anchor. The working suture has a first end operatively connected to at least one of the suture anchor, the first suture portion and the second suture portion. Optionally, the device is arranged such that the first suture portion and the second suture portion are part of a single two-tailed suture.

In a fourth aspect, a device securing soft tissue to bone includes a suture anchor, a first suture, and a second suture. The suture anchor is capable of being positioned within a bone hole. The suture anchor includes a first end, a second end, an outer wall extending along a length between the first end and the second end, and a hollow interior extending along at least a portion of the length. The first suture includes a hollow braid. The first suture passes through the outer wall of the suture anchor at least once. The first suture includes an internally disposed portion positioned within the hollow interior of the suture anchor. The second suture has a body extending from a first end portion to a second end portion. At least part of the first end portion is coupled to the first suture. At least part of the body passes internally through the hollow braid of the first suture. The second suture is entirely external to the suture anchor.

Optionally, the first suture includes an exteriorly disposed portion that encircles the suture anchor circumferentially around the outer wall at least one half of a complete rotation, the at least part of the body of the second suture passing internally through the hollow braid of the first suture in the exteriorly disposed portion of the first suture.

Optionally, the first suture comprises an exteriorly disposed portion and a snail portion on opposite sides of the internally disposed portion, the snail portion having a first sub-portion external to the outer wall and a second sub-portion internal to the outer wall.

In a fifth aspect, a device for securing soft tissue includes a suture anchor, a first suture and a second suture. The suture anchor is capable of being positioned within a bone hole and includes a first end, a second end, an outer wall extending along a length between the first end and the second end, and a hollow interior extending along at least a portion of the length. The first suture includes a hollow braid and passes through the outer wall at a first location on the suture anchor and at a second location on the suture anchor separate from the first location. The second suture is movably disposed relative to the first suture such that at least part of the second suture is capable of passing internally through the hollow braid of the first suture, and the second suture is always entirely external to the suture anchor. Optionally, the device is configured such that one end portion of the first suture is attached to the second suture and the first suture is capable of passing through the hollow braid of the fist suture when the second suture is pulled out of the hollow braid of the first suture, the first suture passing through the same part of the hollow braid as the second suture. Optionally, the device is arranged so that the first suture passes through the outer wall at a third location in between the first location and the second location, and the second suture passes internally through the hollow braid of the first suture between the first and second locations and between the second and third locations.

In a sixth aspect, a device for securing soft tissue includes a suture anchor, a suture and a shuttle. The suture anchor includes a channel extending at least partially therethrough and a movable piece disposed in the channel. The suture includes an enlarged first end and is at least partially disposed in the channel such that the moveable piece separates the enlarged first end from a remainder of the suture. The suture has a hollow braid and the shuttle is capable of passing internally through a portion of the hollow braid. Prior to deployment of the device, a portion of the shuttle is disposed internally through the portion of the hollow braid. The device is further configured such that when a first end portion of the shuttle is attached to the suture and a second end portion of the shuttle opposite the first end portion of the shuttle is pulled, the enlarged first end of the suture is pulled toward a trailing end of the suture anchor and the enlarged first end of the suture pushes the movable piece within the channel of the suture anchor, causing the suture anchor to expand in a radial direction. While the moveable piece moves from an initial position within the channel to a deployed position within the channel closer to the trailing end of the suture anchor, the portion of the suture moves from a first location within the channel of the suture anchor to a second location outside of the suture anchor, and a second end of the suture opposite the enlarged first end passes through the portion of the suture. Optionally, the suture anchor may be made of a material such that the suture anchor is inflexible relative to the suture. Similarly, the material of the suture anchor may have a significantly lower porosity than the suture. Optionally, the suture anchor may be made of a biocompatible plastic such as polyether ether ketone, an absorbable polymer, such as poly-L-lactic acid, bio-active materials such as hydrogels, or a metal, such as stainless steel or titanium.

In a seventh aspect, a device for securing soft tissue to bone includes a suture anchor, a first suture and a second suture. The suture anchor is capable of being positioned within a bone hole and includes a first end, a second end, an outer wall extending along a length between the first end and the second end, and a hollow interior extending along at least a portion of the length. The first suture includes a hollow braid and passes through the outer wall of the suture anchor at least once. The first suture also includes an internally disposed portion positioned within the hollow interior of the suture anchor. The second suture has a body extending from a first end portion to a second end portion, at least part of the first end portion being coupled to the first suture and at least part of the body passing internally through the hollow braid of the first suture. The second suture is entirely external to the suture anchor.

Optionally, the device may be arranged so that the first suture includes an exteriorly disposed portion that encircles the suture anchor circumferentially around the outer wall at least one half of a complete rotation, the at least part of the body of the second suture passing internally through the hollow braid of the first suture in the exteriorly disposed portion of the first suture. Optionally, the device may be arranged so that the first suture also includes an exteriorly disposed portion and a snail portion on opposite sides of the internally disposed portion, the snail portion having a first sub-portion external to the outer wall and a second sub-portion internal to the outer wall.

In an eighth aspect, the present disclosure relates to a method of securing soft tissue to bone. The method commences with obtaining a device. The device obtained may be any one of the devices described herein. In a first example of the method, the device is that described in the second aspect. In this manner, the initial step of the method involves obtaining a device that includes a suture anchor, a first suture, a shuttle and a second suture. The suture anchor is capable of being positioned within a bone hole and includes a first end, a second end, an outer wall extending along a length between the first end and the second end, and a hollow interior extending along at least a portion of the length. The first suture includes a first end portion, a second end portion, and a hollow braid. The first suture passes through the outer wall of the suture anchor at least once. A portion of the first suture is positioned within at least a portion of the hollow interior of the suture anchor and another portion of the first suture encircles the suture anchor circumferentially around the outer wall at least one half of a complete rotation, such as at least one complete rotation. The shuttle includes a length and a suture-engaging structure and is capable of being positioned within the hollow braid of the portion of the first suture encircling the suture anchor. The second suture includes a first end portion and a second end portion. One of the first or second end portions of the second suture is coupled to one of the first or second end portions of the first suture, while the other of the first or second end portions of the second suture is capable of being coupled to the suture-engaging structure. Additionally, the shuttle is capable of being actuated to move the other of the first or second end portions of the second suture into the hollow braid of the first suture.

After obtaining the device, the method proceeds with the following steps: positioning the suture anchor in bone; passing the other of the first or second end portions of the second suture around or through the soft tissue to be secured; coupling the other of the first or second end portions of the second suture, passed around or through the soft tissue to be secured, with the suture-engaging structure; actuating the shuttle to move the other of the first or second end portions of the second suture into the hollow braid of the first suture; and tensioning the other of the first or second end portions of the second suture, positioned through the hollow braid of the first suture, to tension the soft tissue against the bone.

It will be appreciated that any of the variations, aspects, features, and options described in view of one of the devices apply equally to the other devices and methods, and vice versa. It will also be clear that any one or more of the above variations, aspects, features, and options can be combined.

### BRIEF DESCRIPTION OF THE DRAWINGS

FIGs. 1A-1B illustrate a soft tissue securement device according to one embodiment of the disclosure.
FIG. 2A illustrates a soft tissue securement device according to one embodiment of the disclosure.
FIGs. 2B and 2C illustrate variations of the soft tissue securement device of FIG. 2A.
FIG. 3 illustrates a variation of the soft tissue securement device according to some embodiments of the disclosure.
FIG. 4 illustrates a variation of the soft tissue securement device according to some embodiments of the disclosure.
FIGs. 5, 6, 7, 8, 9, 10, 11, 12 and 13 illustrate a soft tissue securement device according to respective embodiments of the disclosure.
FIG. 14A illustrates a variation of the soft tissue securement device according to some embodiments of the disclosure.
FIG. 14B illustrates another variation of the soft tissue securement device according to some embodiments of the disclosure.

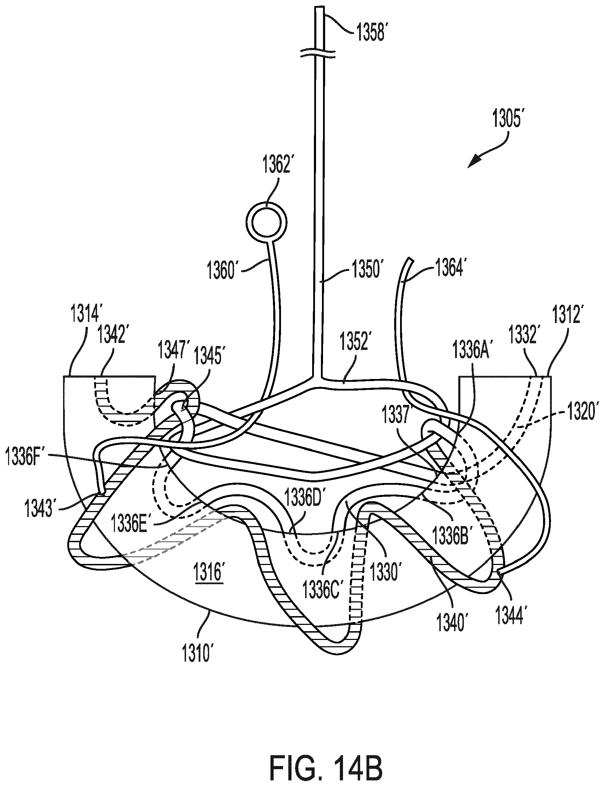

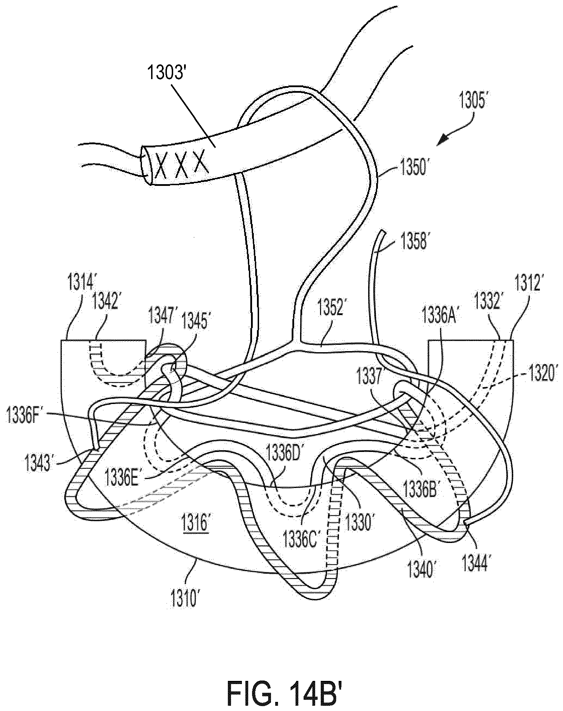

FIG. 14B' illustrates the soft tissue securement device of FIG. 14B with an adjustable loop.

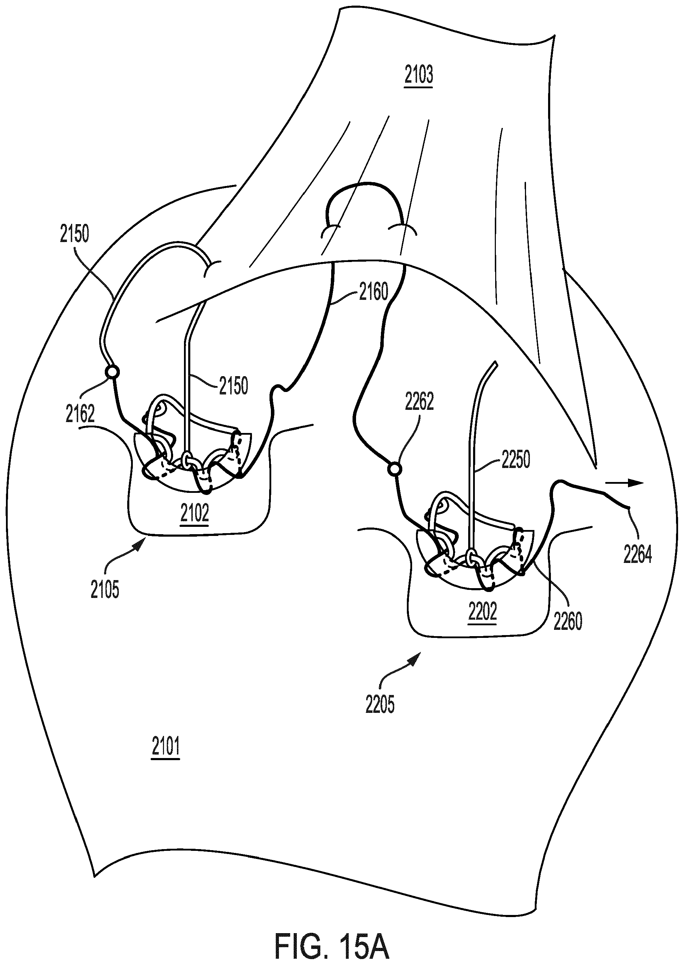

FIGS. 15A-B illustrate exemplary arrangements of two securement devices according to some embodiments of the disclosure.

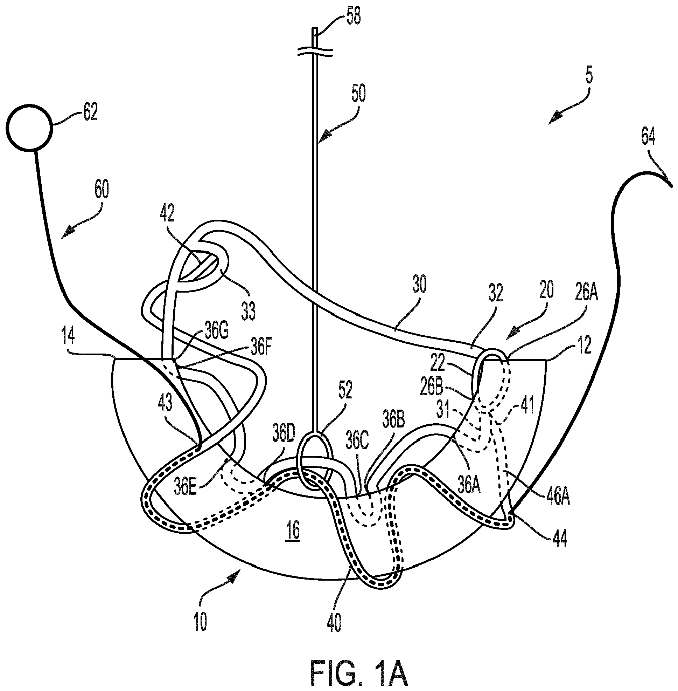

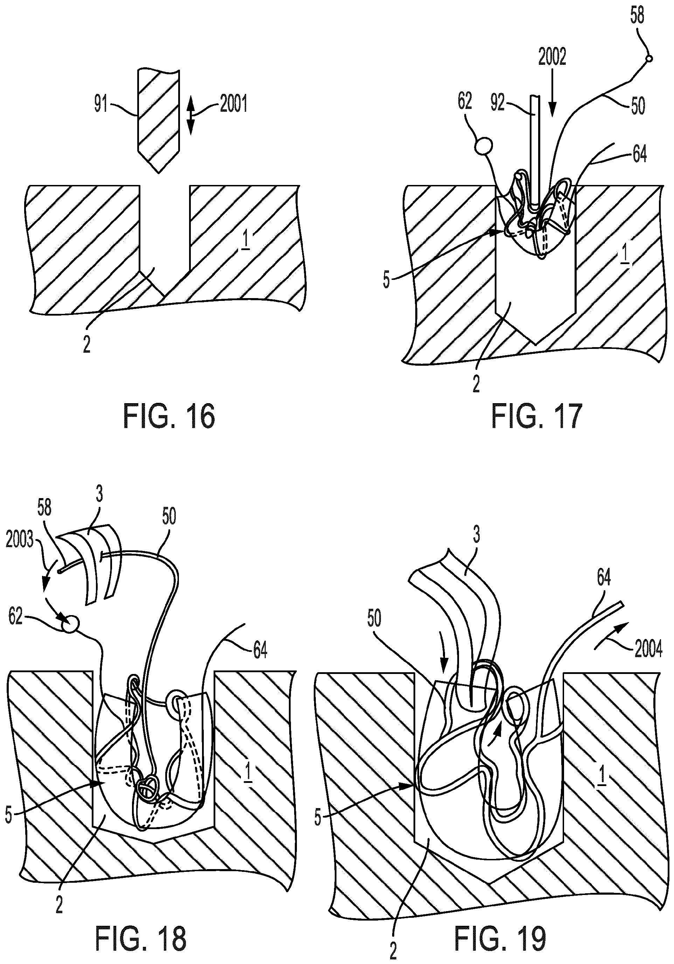

FIGs. 16-19 describe steps in a method of using the soft tissue securement device of FIG. 1A according to one embodiment of the disclosure.

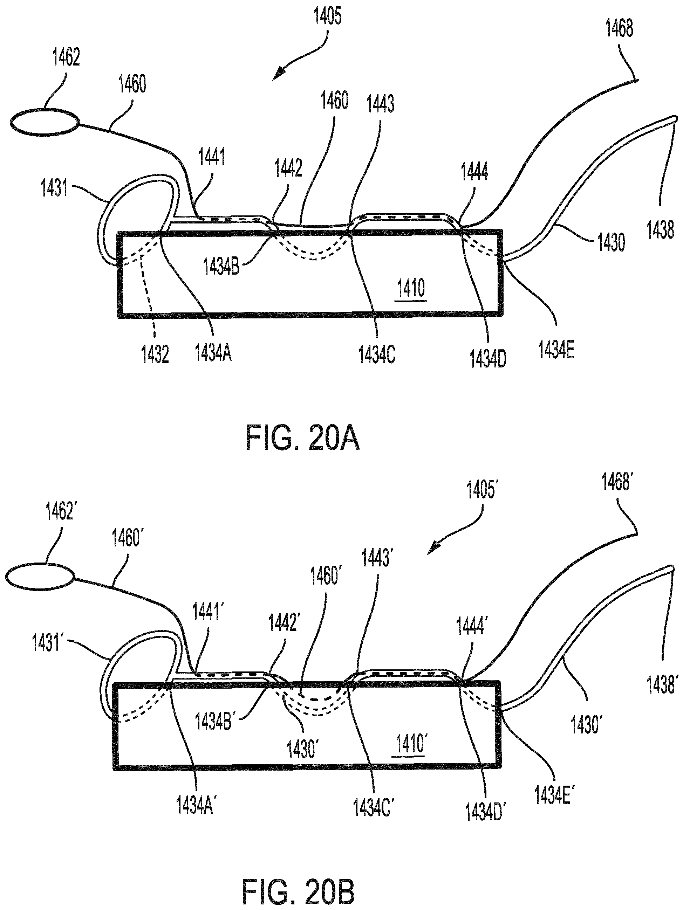

FIG. 20A illustrates a soft tissue securement device according to one embodiment of the disclosure.
FIG. 20B illustrates a variation of the soft tissue securement device of FIG. 20A.

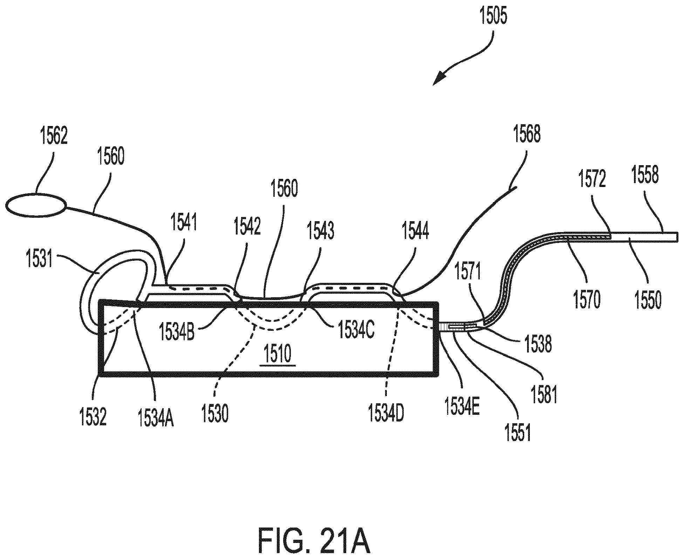

FIG. 21A illustrates a soft tissue securement device according to one embodiment of the disclosure.

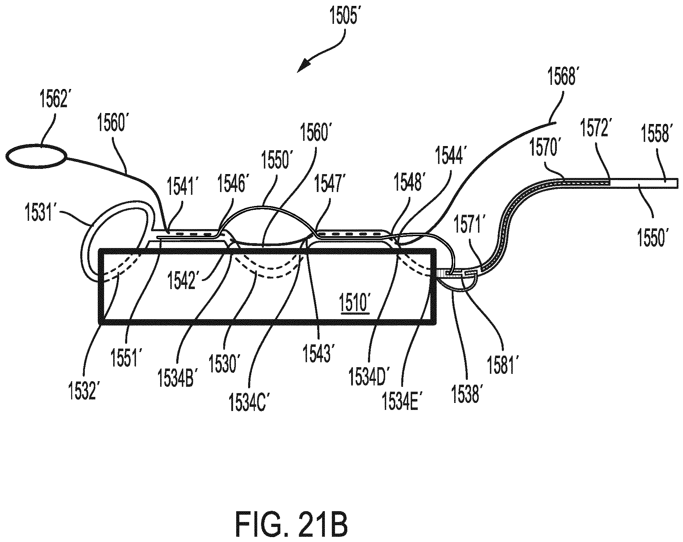

FIG. 21B illustrates a variation of the soft tissue securement device of FIG. 21A.

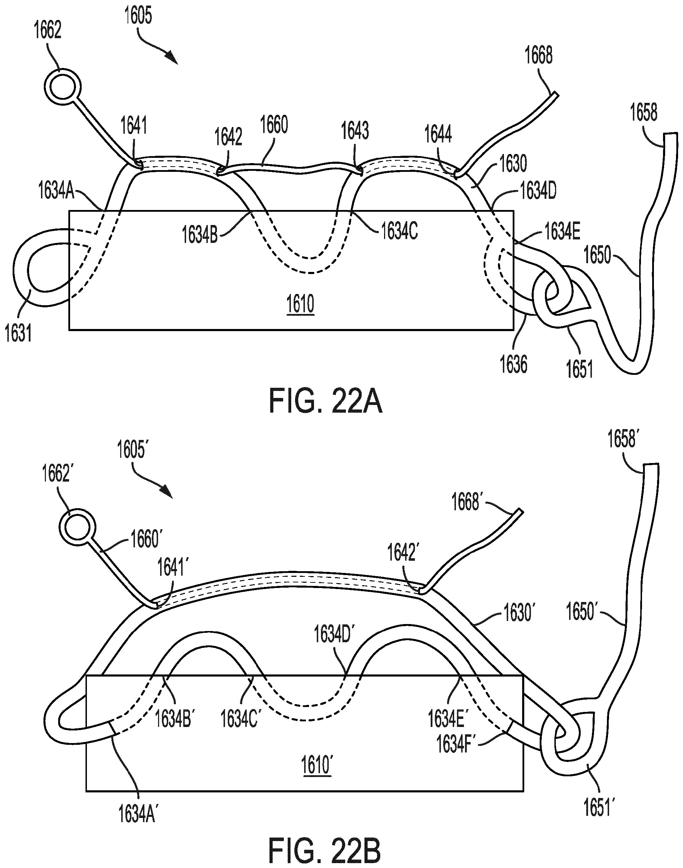

FIG. 22A illustrates a soft tissue securement device according to one embodiment of the disclosure.
FIG. 22B illustrates a variation of the soft tissue securement device of FIG. 22A.

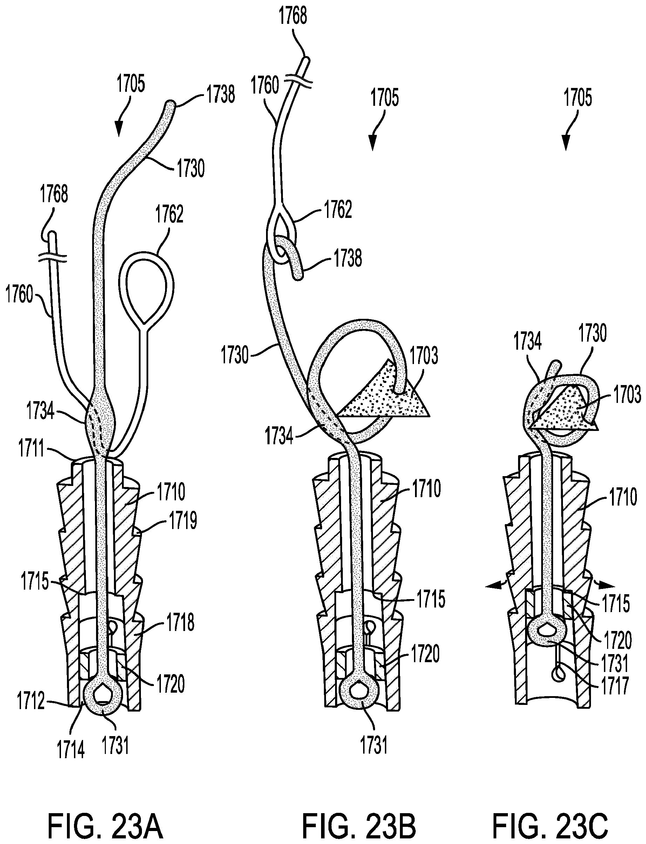

FIGs. 23A-C illustrate a soft tissue securement device and steps in a method of using the soft tissue securement device according to some embodiments of the disclosure.

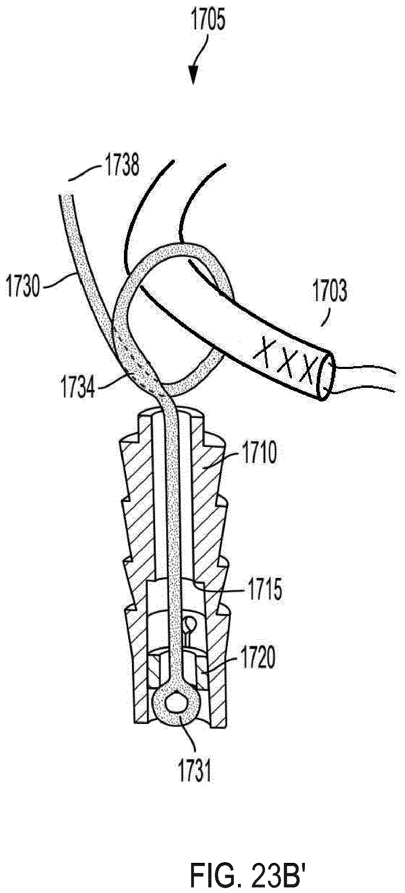

FIG. 23B' illustrates the soft tissue securement device of FIGs. 23A-C with an adjustable loop.

### DETAILED DESCRIPTION

The present disclosure describes various embodiments of suture securement devices, systems and associated methods intended for use in tissue, such as bone or soft tissue. Soft tissue may be, for example, meniscus, cartilage, ligaments and tendons, or the like. While many of the exemplary methods disclosed herein are directed towards use of a suture securement device such as a suture anchor for implantation into a bone hole, with the suture anchor securing tissue thereto, other uses, some of which are described herein, are also envisioned. Exemplary surgical applications include rotator cuff repair, shoulder labrum repair and hip labrum repair. As used herein, "proximal" or "proximally" means closer to or towards an operator, e.g., a surgeon, while "distal" or "distally" means further from or away from the operator.

The various embodiments disclosed herein generally utilize two or more lengths of suture and one or more suture anchors. The suture may be a typical suture generally known in the art, and specifically the orthopedic surgery art, such as braided sutures. A braided suture may be formed by braiding or weaving multiple strands together. The strands, in turn, may be formed by combining multiple filaments. In some examples, multiple filaments may be twisted together to form a strand. To avoid ambiguity, such braided sutures may be formed from a series of braided or woven strands, where each strand is formed from a series of filaments. Sutures contemplated by the present disclosure may be substantially hollow or substantially solid depending on the application of the suture included in a particular embodiment. In other words, a suture is at least substantially hollow, as used herein, in that it has some amount of an interior volume along its length in which another suture portion (whether of the same suture or another suture), or other material, may be positioned within the braid; one such example is a braided multifilament suture where the braided strands form an outer sheath surrounding an inner core and/or an interior volume. Alternatively, a solid suture does not include any interior volume within the suture where another suture portion could be positioned; one such example is a monofilament, as well known in the art, while another example may be a flat suture tape which is braided as a flat sheet. The sutures may have a substantially round (e.g., cylindrical) or substantially flat (*e.g.,* tape) shape. Further, stiffness in the suture may be varied. The suture may include, for example, a relatively stiff portion, relative to the rest of the suture, which can provide beneficial uses such as simpler threading through small devices, such as a fixation device like a ReelX suture anchor (Stryker Endoscopy, San Jose, CA), or to provide a stiff portion which may be pushed through a cannula or other instrument, such as a suture passer. Similarly, the suture may include one or more ends that are tapered which may provide beneficial uses in terms of passing the smaller suture end through tissue, through a device such as a suture anchor, and/or through a body of a suture (or itself) such as in the creation of a splice, finger trap, or the like. The suture may be constructed of synthetic material, such as ultra-high molecular weight polyethylene (UHMWPE), poly lactic-co-glycolic acid (PLGA) or the like, or of organic material (silk, animal tendon, or the like).

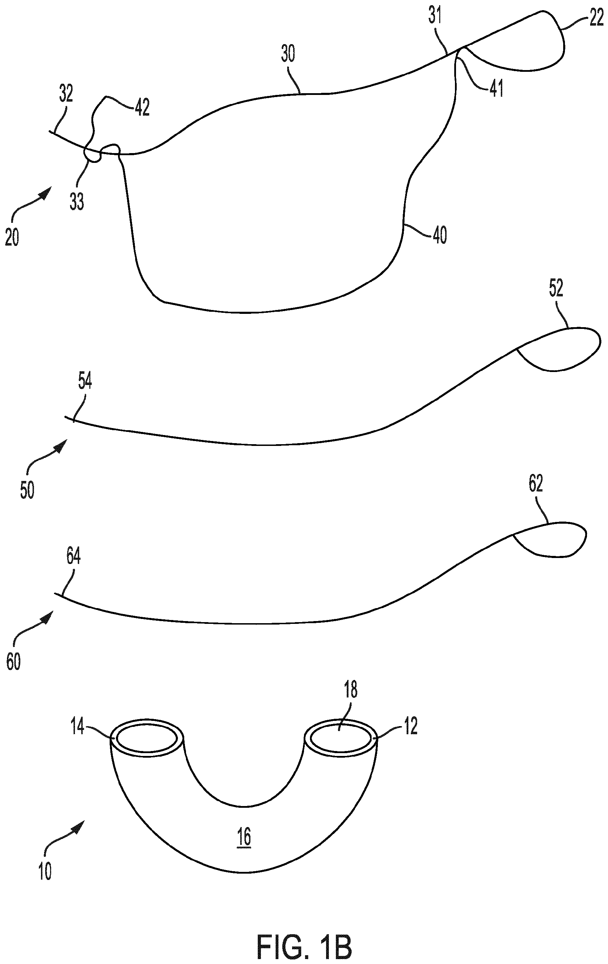

The sutures of the present disclosure may also be modified to include loops and splices. Such suture may include one or more loops formed along its length (see e.g., loop portion 22 of first suture 20 in FIGs. 1A and 1B). Such loops are typically formed by splices within the braid structure of the suture or by forming the loop during the braiding manufacturing step itself, which may also involve splices, though other methods of forming, such as simply by pulling, tearing or cutting the braid structure, or by tying a loop using a knot or the like, are also possible as known in the art. Still further, the loop could be made by bifurcating the braid structure during the braiding manufacturing. Examples of splices that may be prepared using the contemplated sutures include brummel splices, tuck splices, eye splices, and chain-link splices. The preparation of each of these splices on a suture may be accomplished based on the knowledge of a person having ordinary skill in the art, and by a variety of techniques known in the art, but to avoid ambiguity, some illustrative examples are provided herein. A Brummel splice may be formed by initially taking a first free end of a suture and passing it through a first opening defined by a space between strands of the same suture. Such step may optionally then be followed by taking a second free end of the suture and passing it through a second opening adjacent to the first opening. And, after that, taking the first free end or the second free end and passing it back through a third opening adjacent to the second opening; the other of the first free end and second free end can then be passed back through a fourth opening adjacent at least one of the other openings. These last step(s) may alternatively involve advancing the first free end and/or second free end into the lumen of the suture through the third opening and/or fourth opening, respectively. Once the suture passes are completed, the end or ends are pulled to secure a closed loop. A tuck splice involves taking an end portion of a hollow suture and passing it back into a more proximal region of the same suture via an opening in the suture so that the end portion is advanced through a lumen of the hollow structure in a coaxial fashion; this may also be referred to as the aforementioned finger trap construct. A chain-link splice involves using known splicing techniques to combine an eyelet or an eye splice on each of two separate sutures. Such sutures may be connected by passing one suture through the other during the formation process. A completed chain-link splice includes two or more loops immediately adjacent to one another so that the suture has the appearance of a "chain-link."

With continued reference to the nature of the sutures contemplated by the present disclosure, the length of suture typically has two free ends (though a suture could be bifurcated or otherwise constructed to have more than two free ends, as is the arrangement for certain sutures in embodiments of the present disclosure, like for example, a so-called two-tailed loop) - the free ends may be separate from one another (as with second suture 50 in FIG. 1A, for example) or may be joined together (as with first suture 20 in FIG. 1A, for example), whether by adhesive, a separate stitch, or formed this way during construction of the braid, or the like. Where a suture has ends that are ultimately joined, the suture may be provided to the user with the free ends separate from one another or joined together. Thus, while some of the exemplary embodiments described herein may use sutures with free ends joined together, and while other of the exemplary embodiments described herein may use sutures with free ends that are separate from one another, any of the exemplary embodiments could start with either of these types of sutures depending on the particular application, anatomical location, surgical procedure (e.g., open versus arthroscopic), and other factors.

The suture anchors used in the present disclosure may be any known in the art, and may be hard (e.g., formed of PEEK or the like) or soft (e.g., all-suture suture anchors such as ICONIX^{®} (Stryker Corporation, Kalamazoo, MI) as shown and described in U.S. Pat. No. 9,445,803, the entire disclosure of which is incorporated by reference herein). The suture anchor may be made from braided strands such that there are gaps between strands or such that strands may be separated to create gaps for the passage of suture through the gaps. Additionally or alternatively, the suture anchor may be formed to define a channel therein to aid in the passage of suture therethrough, much like ICONIX^{®} includes. Other soft anchors are also envisioned, such as where the anchor is formed of braided suture material in the form of flat tape suture material, as known in the art, or of a complete circle of either flat tape or circular and substantially hollow suture material. The suture anchor may include an eyelet, cannulation, opening, aperture, or other structure to allow one or more sutures to be connected thereto. It should be understood that when a suture is coupled or anchored to a suture anchor in any embodiment throughout the present disclosure, the suture may be passed through any portion of the anchor, such as an entire length of the suture anchor, an aperture located anywhere on the anchor. For example, first suture 20 may be passed through suture anchor 10 from a first end 12 to a second end 14, as shown in FIG. 1A.

In one aspect, the present disclosure relates to a soft tissue securement device for use in anchoring tissue on a patient, where such tissue may be in a j oint. In one embodiment, the securement device is as shown in FIG. 1A and indicated by reference numeral 5. Securement device 5 includes a suture anchor 10, a first suture 20, a second suture 50 and a shuttle 60. These components are shown together in FIG. 1A and individually in FIG. 1B.

Suture anchor 10 has a length that extends from a first end 12 to a second end 14 and, as shown, has an external surface 16 between its ends that defines a semi-circular shape, although other shapes are contemplated with a view to shapes suitable for receipt in a bone hole for anchorage. Further, the suture anchor is hollow such that an internal surface 18, visible in part in FIG. 1B, defines a lumen, i.e., a hollow interior that extends from first end 12 to second end 14.

First suture 20 includes a loop portion 22, a snail extension 30 and a boa extension 40, where the snail and boa extensions extend from the loop portion, as shown in FIG. 1B. In the embodiment shown in Figures 1A and 1B, first suture 20 also includes a snail loop 33 on the snail extension. One or both of loop portion 22 and snail loop 33 may be formed via a Brummel splice or a tuck splice, for example. First suture 20 is formed on suture anchor 10 as shown in FIG. 1A. Loop portion 22 includes a first segment external to suture anchor 10 and a second segment passing into the lumen of the suture anchor from locations 26A and 26B located along the length of the loop portion. In some examples, loop portion 22 passes through external surface 16 of suture anchor 10 at least at one location and is otherwise external to suture anchor 10. In such examples, boa extension 40 may be entirely external to anchor body 10 by abutting loop 22 at a location external to external surface 16. In the embodiment shown, entry of loop portion 22 into suture anchor 10 at location 26B may be through a preestablished gap on the suture anchor surface or through a space created between strands or filaments that define suture anchor 10. Entry at location 26A may be through an existing opening at first end 12 of suture anchor 10 or through a space created between strands of the suture anchor. In a variation of securement device 5, snail extension 30 of first suture 20 may singularly pass through external surface 16 at location 36A and then out of the anchor at location 26A, becoming boa extension 40 (or in yet another variation, snail extension 30 could pass out of the anchor at location 26B and then become boa extension 40; or in yet another variation, location 26A could also be on external surface 16 such that suture 20 passes through external surface 16 instead of out of first end 12 as illustrated in FIG. 1A). In this arrangement, there is no loop 22 (as in FIG. 1A) formed using first suture 20. Rather, portions of the snail and boa pass over each other, but are not connected, spliced or attached in any way. In this manner, single first suture 20 has the appearance of a loop from the view shown in FIG. 1A, but there is no splice or knot in the part of the first suture between the boa extension 40 and location 36A. It should be appreciated that this design variation excluding a loop formed via a splice may be substituted for the loop feature in any embodiment herein where a two-tailed loop such as that shown in FIG. 1B is contemplated.

Snail extension 30 extends from a connection end 31 at loop portion 22 to a free end 32, as shown in FIG. 1B. During manufacture or at another time up to commencement of surgery, free end 32 may be attached to loop portion 22 as shown in FIG. 1A through a splice, knot or other means known to persons of ordinary skill. In some variations, free end 32 may be attached to suture anchor 10 or another part of snail extension 20. In other variations, snail extension 30 may pass out of external surface 16 at location 36G and have a terminal end at a minimal distance from location 36G. For example, snail extension 30 may end proximal to location 36G. In further examples, a free end 42 of boa extension 40 may be spliced or knotted to the end of the snail extension at such terminal end of snail extension proximal to location 36G. Further details of boa extension 40 are provided below. Snail extension 30 is arranged to pass in and out of the lumen of anchor body 10 between connection end 31 and free end 32, as shown in FIG. 1A. In the particular arrangement shown, snail extension 30, describing its position from connection end 31 to free end 32, passes out of external surface 16 of the suture anchor at location 36A, passes back into the suture anchor at location 36B, out at location 36C, back in at location 36D, out at location 36E, back in at location 36F, then out at location 36G. In some examples, one or more of the passes through external surface 16 at locations 36A-36G is through a gap created by separating strands or filaments that define the suture anchor. In other examples, the suture anchor may include holes formed during the fabrication process for passage of the snail extension. It should be appreciated that in variations of the securement device similar to the depicted embodiment, the snail extension may pass into and out of the suture anchor a smaller or greater number of times than that shown in FIG. 1A.

Boa extension 40 extends from a connection end 41 to a free end 42, as shown in FIG. 1B. At least a portion of a length of boa extension 40 is hollow. During manufacture or at another time up to commencement of surgery, free end 42 may be connected to snail loop 33 as shown in FIG. 1A through a splice, knot or other means known to persons of ordinary skill. Boa extension 40 is arranged to pass out of the suture anchor close to first end 41, then continue toward an opposite end of the suture anchor by repeatedly encircling external surface 16 as shown in FIG. 1A. In particular, boa extension 40, describing its position from connection end 41 to free end 42, passes out of suture anchor 10 at location 46A, then, positioned external to the exterior surface of the suture anchor, encircles the suture anchor in a forward direction such that each complete encirclement brings boa extension 40 closer to second end 14 of the suture anchor. In the embodiment as depicted, boa extension 40 completely encircles suture anchor 40 two times from location 46A to a location where a remainder of its length to free end 42 extends directly to snail loop 33 for securement thereto, as shown in FIG. 1A. It should be appreciated that while in some examples, the boa extension encircles the suture anchor two or more complete rotations, in other examples, the boa extension encircles the suture anchor at least one complete rotation but less than two complete rotations. Still further, in some examples, a boa extension may encircle the suture anchor one complete rotation or less than one complete rotation, such as for example, the boa extension encircles the suture anchor at least one half of a complete rotation but less than a complete rotation.

As mentioned above, first suture 20 is formed with suture anchor 10 so that loop portion 20 is formed to pass through the external surface of the suture anchor. Second suture 50 includes a length extending from a suture engagement structure 52 to a free end 58 opposite the suture engagement structure. Suture engagement structure 52, shown as an eyelet in FIG. 1A, is formed in the depicted embodiment such that both snail extension 30 and boa extension 40 pass through and are encircled by such structure 52 as they pass outside of suture anchor 10, thereby being operatively connected to the first suture. Thus, as illustrated, second suture 50 is operatively connected to snail extension 30 and boa extension 40. In some examples, such as the arrangement as shown in FIG. 1A, none of second suture is positioned within the hollow interior of suture anchor 10. In other examples, second suture 50 is operatively connected to snail extension 30, and/or boa extension 40 and/or to suture anchor 10, and may also be fabricated in conjunction with the first suture and the suture anchor. In still further examples, shuttle 60, described in greater detail below, may pass externally out of the lumen of boa extension 40 over a distance along a portion of the boa extension wrapping around the suture anchor. In these examples, shuttle 60 may, optionally, pass through suture engagement structure 52. Additionally, while second suture is shown connected to the first suture between locations 36C and 36D, it should be appreciated that second suture may be attached at any location along a portion or portions of first suture and/or on the suture anchor.

Shuttle 60, as with the second suture, has a length extending from a suture engagement structure 62 to a free end 64, as shown in FIGs. 1A and 1B. Suture engagement structure 62 may be a suture loop, as illustrated, or may be a hollow suture end that can form a finger trap connection with another suture end, a Nitinol wire loop, or the like. As incorporated into securement device 5, shuttle 60 is passed through a hollow portion of boa extension 40, as shown in FIG. 1A. In particular, free end 64 is passed through an entry opening 43 in the boa extension, through the lumen within the boa extension, and then out of exit opening 44. Each opening 43, 44 may be formed through separation of strands that form the boa extension or such openings may be created during the initial fabrication process. As described in greater detail in the methods of the present disclosure, such an arrangement of sutures allows for the creation of a one-way tensioning mechanism, such as a finger trap, when the shuttle pulls the second suture into and through the lumen in the first suture.

One advantage of the securement device involves the inclusion of a snail suture and a boa suture. This may be derived from, for example, a first suture including a snail extension and a boa extension, or, in some arrangements, separate standalone snail and boa sutures that may be connected to one another. By having a snail suture and a boa suture arranged relative to a suture anchor as contemplated by the present disclosure, the snail suture may physically support the boa suture and prevent the boa suture from sagging, separating or otherwise drifting too far from the suture anchor external surface. Thus, during use of the securement device in a method of anchoring soft tissue, the boa suture will not separate or move from the suture anchor to any detrimental extent as the one-way tensioning mechanism, e.g., finger trap, is created through the pulling of one or more shuttles. In other words, the snail suture may help keep the boa suture in place during tensioning of the shuttle 60 and second suture 50 to form the one-way tensioning mechanism.

One such structure intended to keep the boa suture from sagging or separating with respect to the suture anchor includes a connection of the boa suture to the snail suture in a region of suture anchor end 14. The connection may be between free end 42 of boa and a portion of the snail extending from the anchor, such as in FIG. 1A the portion extending from location 36G at anchor end 14. In this manner, snail suture 30, through its passage into and out of the suture anchor, limits an extent that boa suture 40 may move away from the suture anchor, keeping boa extension 40 close to anchor body 16. In contrast, if the boa suture were not connected to the snail suture, the boa suture may move away from the suture anchor because the boa suture would only be held in place on loop 22 end of the first suture. Some circumstances where this could occur include when the sutures are at rest on the suture anchor and when tension is applied while the securement device is being secured in place in a bone. For example, when the second suture is tensioned, the arrangement of securement device 5 prevents the boa suture from moving away from the suture anchor, e.g., prevents it from moving upward out of the implantation site. And, the arrangement of the sutures is such that the pulling of the shuttle through the boa does not cause the boa to be deleteriously drawn toward the leading end 1364 of the shuttle. In variations, the boa suture may pass around and remain external to anchor body 16 in ways other than that shown in FIG. 1A, extending between loop 22 and a free end 42 of boa spliced or knotted to snail extension 30.

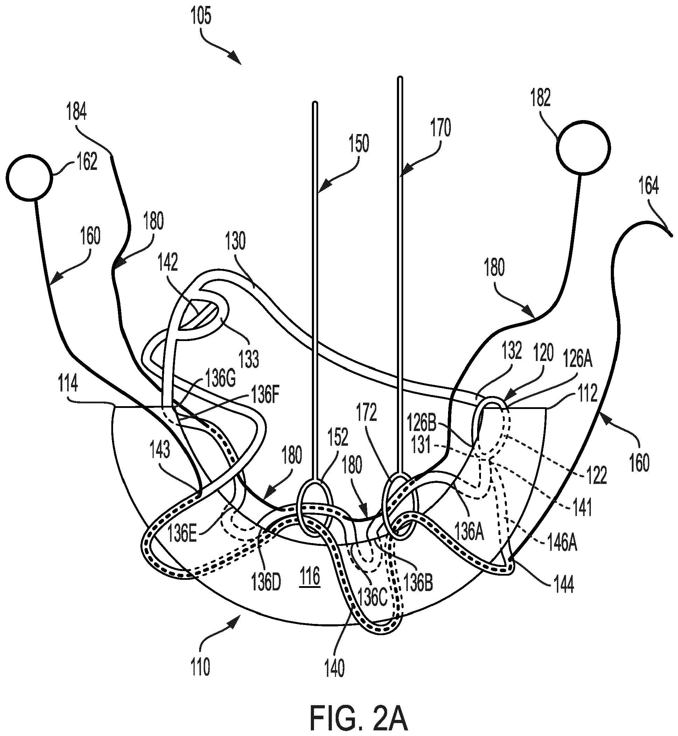

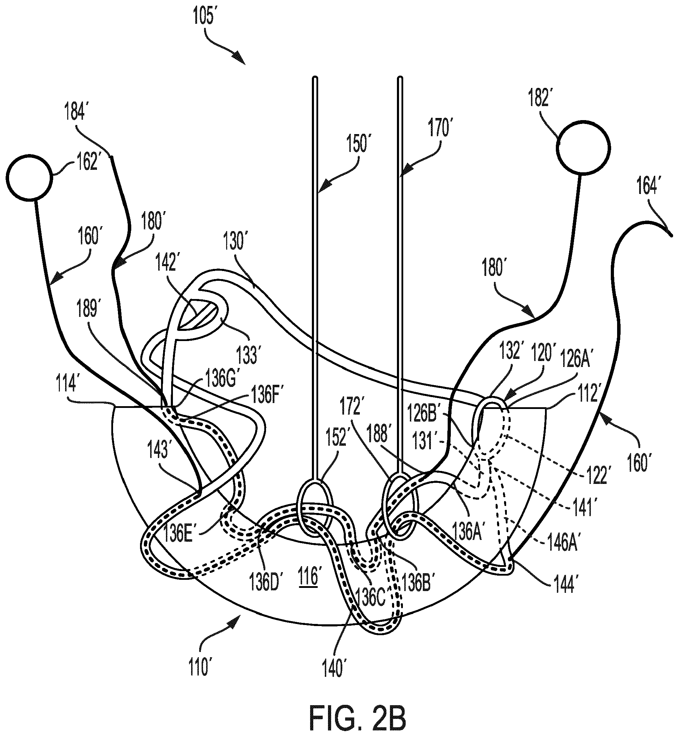

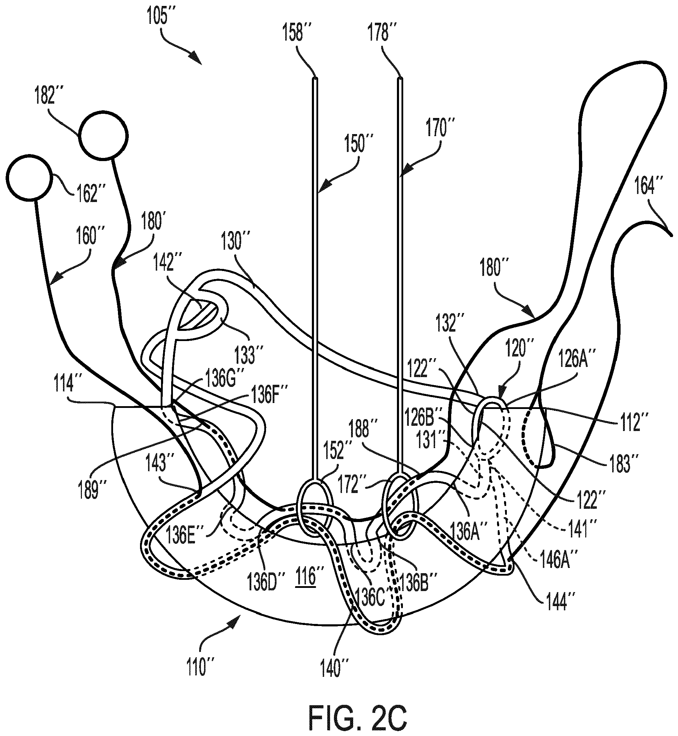

FIG. 2A depicts another embodiment of securement device, indicated by reference numeral 105. In FIG. 2A, the 100 series of reference numerals refer to like elements of the double-digit series of reference numerals in securement device 5 of FIG. 1A, unless otherwise noted. Securement device 105 includes suture anchor 110, a first suture 120, a second suture 150, a third suture 170, a first shuttle 160 and a second shuttle 180. First suture 120 includes a loop portion 122, a snail extension 130 and a boa extension 140. As with the embodiment of FIG. 1A, first shuttle 160 is positioned through a portion of boa extension 140 between entry and exit locations 143, 144. In a different arrangement relative to first shuttle, second shuttle 180 is positioned through one or more separate portions of snail extension 130, each portion being external to suture anchor 10. Specifically, second shuttle 180 passes through the lumen of snail suture 130 in a first portion between points 136A and 136B, a second portion between points 136C and 136D and a third portion between points 136E and 136F, while being located outside of snail extension between these portions. A remaining portion of second shuttle 180 extending from a location proximate point 136A extends to suture engagement structure 182 while another remaining portion of second shuttle 180 extending from a location proximate point 136F extends to free end 184. While suture engagement structure 152 of second suture 150 may be disposed around snail extension 130, boa extension 140, and/or suture anchor 110 in any manner as described for second suture 50, third suture 170 includes a suture engagement structure 172 that may also be disposed in various forms of attachment to one or more parts of first suture 120 and suture anchor 110 as described for second suture 50. Third suture 170 may be disposed at a location one-pass of suture closer to first end 112 of suture anchor 110, as shown in FIG. 2. Although the second and third sutures are depicted at a particular location along a length of suture anchor 110 in FIG. 2A, it should be appreciated that either of second suture anchor 150 or third suture anchor 170 may be attached to first suture 120 and/or suture anchor 110 at any location along the length of the suture anchor, and in any order relative to one side.

In a variation of securement device 105 shown in FIG. 2B and identified by reference numeral 105', the 100' series of reference numerals refer to like elements of the 100 series of reference numerals of FIG. 2A, unless otherwise noted. Securement device 105' includes second shuttle 180' disposed in part through snail extension 130'. In this arrangement, second shuttle 180' is disposed through an entire portion of snail extension between entry and exit points 188', 189', where such points are proximate points 136A' and 136G', respectively. In this way, second shuttle 180' passes through portions of snail extension 130' that are both external and internal to suture anchor 110'. In some examples, entry point 188' and exit point 189' may be at other locations along a length of snail extension 130', while still encompassing a portion of snail extension 130' that passes both externally and internally to suture anchor 110'.

In another variation of securement device 105 shown in FIG. 2C and identified by reference numeral 105", the 100" series of reference numerals refer to like elements of the 100 series of reference numerals of FIG. 2A, unless otherwise noted. Securement device 105" includes second shuttle 180" with a length extending from a loop 183", e.g., an eye splice, at a first end to a suture engagement structure 182" at a free second end. In some examples, loop 183" may pass at least once through external surface 116" of suture anchor 110". While the first end is depicted as an eye splice 183", other forms of anchorage of the first end are also contemplated, as described throughout the present application. In this embodiment, second shuttle 180" functions as a shuttle and a working suture, as is described in greater detail in the methods of the present disclosure. During use, and described in greater detail in the method embodiments, second suture 150" may be passed through soft tissue and then secured to suture engagement structure 182" of second shuttle 180". Then, an opposite end of second shuttle may be pulled to bring a remainder of the second shuttle through snail extension 130" to create a one-way tensioning mechanism using second suture 150". At such time, second shuttle 180" at end 112" of suture anchor may be cut from second suture 150" and then itself passed through another tissue location and attached to suture engagement structure 162". At such time, second shuttle 180" may be used to create a second one-way tensioning mechanism through boa extension 140" by pulling on first shuttle 160". Securement device 105" may also include a third suture 170", though such suture is optional in this variation.

As will be described further in the methods of using the securement device, the configuration of securement device 105, 105', 105" allows for the suture to be anchored to tissue at two separate tissue locations outside of the anchorage point. Specifically, using securement device 105 as an example, anchorage of a first tissue location may be performed using second suture 150 and suture engagement structure 162 on first shuttle 160 and anchorage of a second tissue location remote from the first location may be performed using third suture 170 and suture engagement structure 182 on second shuttle 180. Similarly, in other variations, it is envisioned that sutures 150 and/or 170 may not necessarily be used with structures 162 and/or 182, but instead, a tail end of any suture, such as a suture extending from another suture anchor or from a "luggage-tag" or other connection to a tissue, could be used with suture engagement structure 162 and/or 182. For proper functioning of the finger trap mechanism, it is generally required that both sides 143 and 144 of the finger trap maintain tension by securing anchor 110 and securing suture 150 (or other suture).

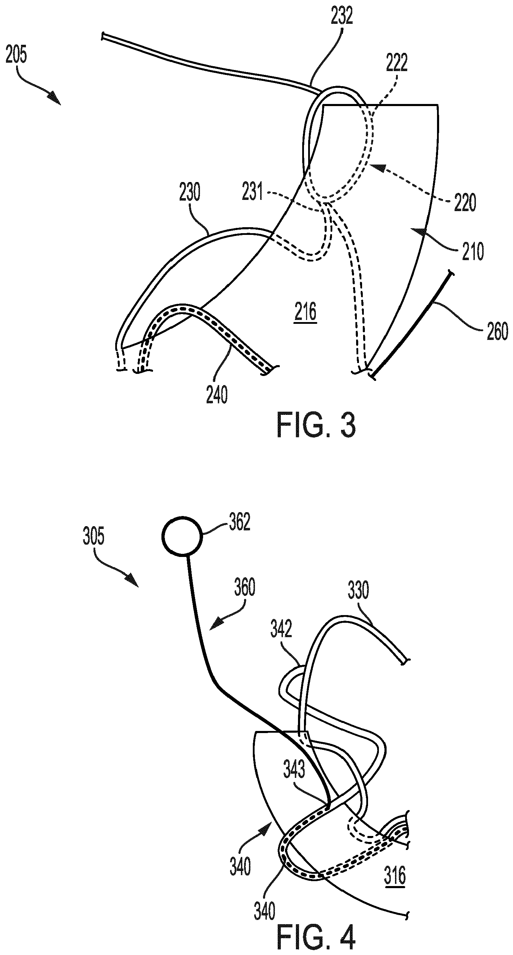

FIGs. 3 and 4 depict still further embodiments. Each of FIGs. 3 and 4 may be variations of other contemplated embodiments, including those depicted in FIGs. 1A, 2, 5-7 and 12-13. FIG. 3 is a partial view of suture anchor 205. In FIG. 3, the 200 series of reference numerals refer to like elements of the double-digit series of reference numerals in securement device 5 of FIG. 1A, unless otherwise noted. In securement device 205, at least three sutures are included. First suture 220 includes loop portion 222 and boa extension 240. Second suture 230 is a snail extension and has a length from first end 231 attached to loop portion 222 to second end 232 also attached to loop portion 222, as shown. In this configuration, the suture defining a snail-type pattern of engagement with suture anchor 210 is standalone second suture 230. The third suture, not shown, may be positioned in a similar manner as second suture 50 shown in FIG. 1A. The remaining features of securement device 205 may be those included in the embodiments depicted in any one of FIGs. 1A, 2, 5-7 and 12-13, for example. In a variation, the loop portion and the snail extension may be a single suture while the boa suture is a separate suture.

FIG. 4 is a partial view of suture anchor 305. In FIG. 4, the 300 series of reference numerals refer to like elements of the double-digit series of reference numerals in securement device 5 of FIG. 1A, unless otherwise noted. In securement device 305, the first suture includes snail extension 330 and boa extension 340, with the boa extension having free end 342. In this configuration, snail extension 330 extends from a first end at a loop portion of the first suture to a free end, passing repeatedly through an exterior surface 316 of suture anchor 310, but without any loops along its length. In this manner, free end 342 of boa extension 340 is directly connected to a portion of a length of snail extension 330, as shown in FIG. 4. A connection of free end 342 to snail extension 330 may be in the form of a splice, knot or other means known to persons of ordinary skill. The remaining features of securement device 305 may be those included in the embodiments depicted in any one of FIGs. 1A, 2, 5-7 and 12-13, for example. In a further variation of FIG. 4, the free end of boa extension is connected to the snail extension so that such connection is disposed within the hollow interior of the suture anchor or embedded on or within the suture anchor.

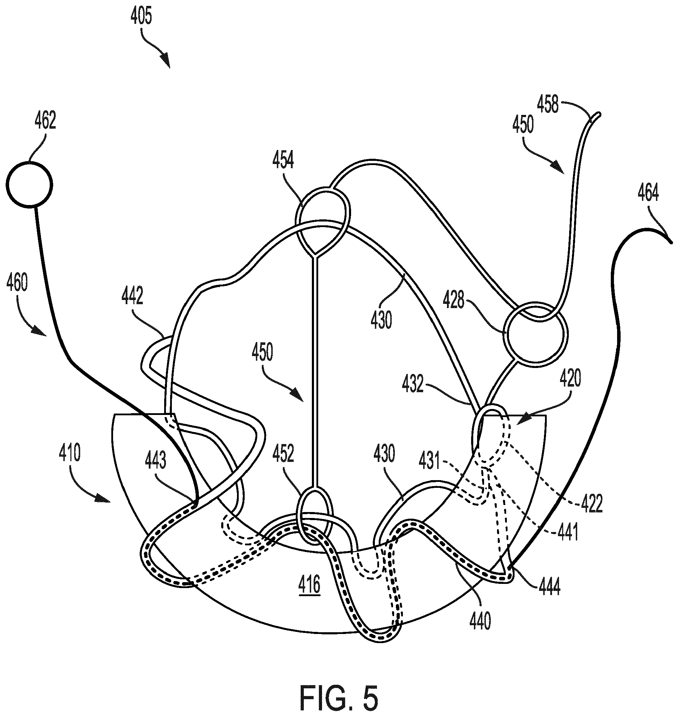

FIG. 5 depicts another embodiment of securement device, indicated by reference numeral 405. In FIG. 5, the 400 series of reference numerals refer to like elements of the double-digit series of reference numerals in securement device 5 of FIG. 1A, unless otherwise noted. Securement device 405, as with securement device 5, includes suture anchor 410, first suture 420, second suture 450 and shuttle 460. In the configuration shown in FIG. 5, first suture 420 includes a free loop portion 428, a suture anchor loop portion 422 passing through surface 416 of suture anchor 410, and snail and boa extensions 430, 440. In a variation, the pair of loop portions on the first suture may be connected with and immediately adjacent to one another to define an eyelet pair. As depicted, a free end 442 of boa extension 440 may join snail extension 430 in a manner similar to that depicted in FIG. 4. Second suture 450 includes an end suture engagement structure 452 and a middle suture engagement structure 454. Second suture is disposed so that snail and boa extensions 430, 440 may pass through end suture engagement structure 452, but may be disposed in other ways as described for securement device 5, while a portion of snail extension 430 closer to free end 432 passes through middle suture engagement structure 454. Further, a portion of second suture 450 between engagement structure 454 and a free end 458 of second suture passes through free loop portion 428 of first suture 420.

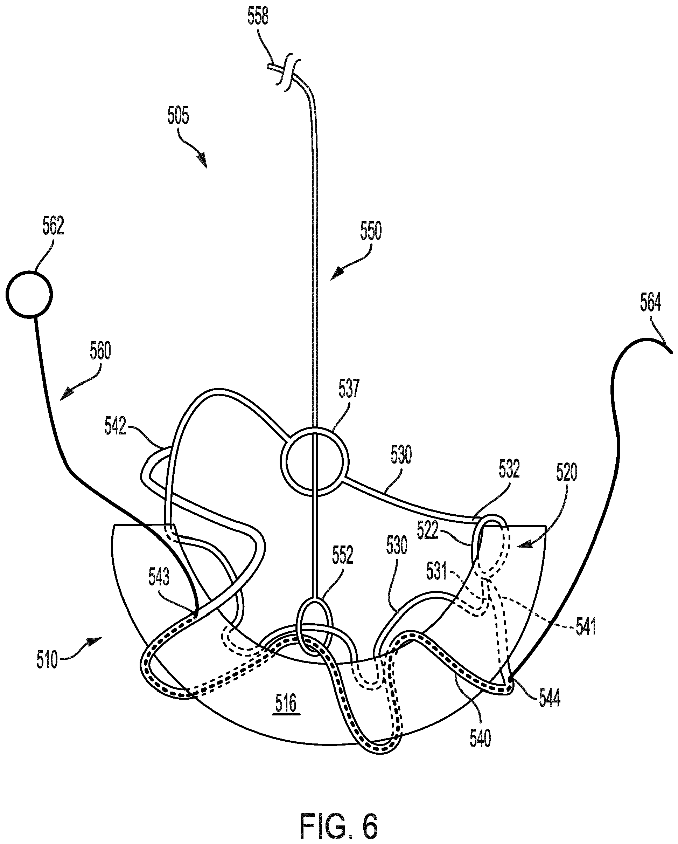

FIG. 6 depicts another embodiment of securement device, indicated by reference numeral 505. In FIG. 6, the 500 series of reference numerals refer to like elements of the double-digit series of reference numerals in securement device 5 of FIG. 1A, unless otherwise noted. Securement device 505 includes suture anchor 510, first suture 520, second suture 550 and shuttle 560. First suture 520 includes loop portion 522, snail extension 530 and boa extension 540. Snail extension 530 passes in and out of suture as shown in FIG. 6 in a manner similar to that described for securement device 5, and includes a distal loop 537 toward a free end 532. As an assembly ready for use, second suture 550 is disposed so that suture engagement structure 552 is operatively connected to one or more of snail extension 530, boa extension 540 and suture anchor 510 and a portion of a length of second suture 550 between ends of the second suture passes through distal loop 537. As depicted, a free end 542 of boa extension 540 may join with snail extension 530 in a manner similar to that depicted in FIG. 4.

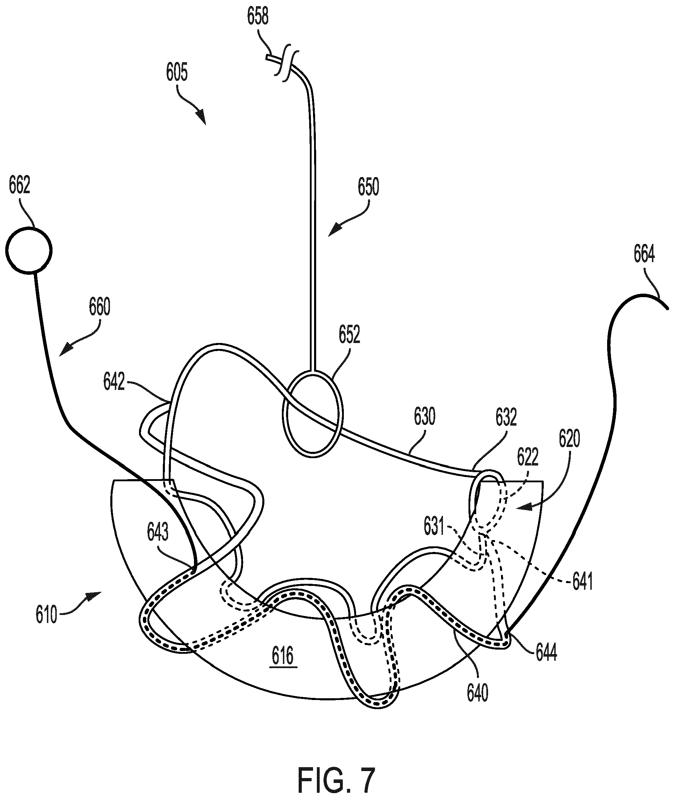

FIG. 7 depicts another embodiment of securement device, indicated by reference numeral 605. In FIG. 7, the 600 series of reference numerals refer to like elements of the double-digit series of reference numerals in securement device 5 of FIG. 1A, unless otherwise noted. Securement device 605 includes suture anchor 610, first suture 620, second suture 650 and shuttle 660. In this configuration, first suture 620 has a similar structure to that of first suture 20 and is disposed relative to suture anchor 610 in a manner similar to that of first suture 20 relative to suture anchor 10. As depicted, a free end 642 of boa extension 640 may join with snail extension 630 in a manner similar to that depicted in FIG. 4. However, suture engagement structure 652 of second suture 650 is disposed so that only a distal portion of snail extension 630 passes through such engagement structure.

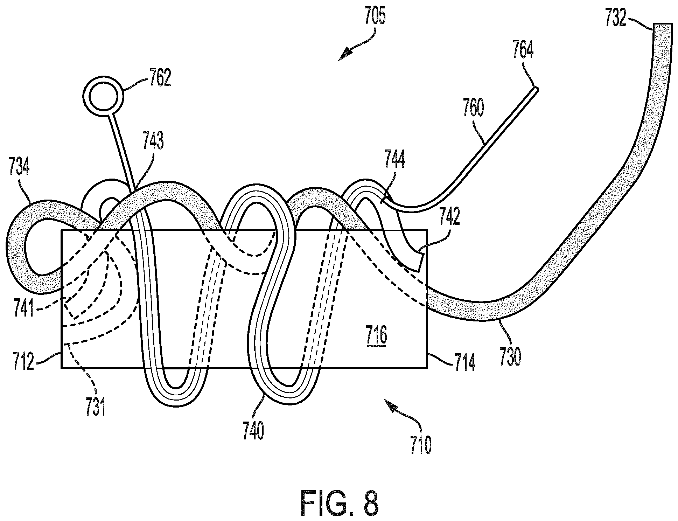

FIG. 8 depicts another embodiment of securement device, indicated by reference numeral 705. Securement device 705 includes suture anchor 710, snail suture 730, boa suture 740 and shuttle 760. Snail suture 730 includes a length extending from an embedded end 731 to a free end 732. Beginning from end 731, the snail suture is spliced through another location on its length to define loop 734, and is positioned so that a portion of loop 734 or a part of the suture on the other side of the loop moving away from end 712 of suture anchor 710 passes through suture anchor 716 or is entirely embedded in the suture anchor. In this manner, a majority of, or in some cases all of loop 734 passes outside external surface 716 of suture anchor 710. Snail suture 730 then extends along a length of the suture anchor while passing in and out of the suture anchor in a direction from end 712 to end 714 until exiting suture anchor at end 714, as shown in FIG. 8. A remaining portion of snail suture 730 external to end 714 extends to free end 732. Boa suture 740 extends from a first end 741 to a second end 742. In some examples, one or both ends, or portions adjacent thereto, are embedded or pass through an external surface 716 of suture anchor 710. In some examples, one or both ends may be tuck spliced into another part of the same suture or may be spliced into the snail suture. Between the ends, boa suture 740 is external to suture anchor 710 and traverses the length of the suture anchor while passing around an outer perimeter of the anchor thereby encircling the anchor while advancing across its length. Boa suture 740 is hollow and includes entry and exit openings 743, 744 for shuttle 760 to pass therethrough, as shown in FIG. 8. Shuttle 760 has a length extending from suture engagement structure 762 to free end 764. In some examples, end 732 of snail suture 730 may have a loop such as an eyelet splice. Such loop may be connected to another loop on the securement device 705 to create an eyelet pair. For example, modified end 732 with a loop may be passed through tissue and connected to loop 734. In such an arrangement, securement device 705 may include a third suture attached to the snail suture, boa suture or suture anchor that is passed through tissue and secured to shuttle end 762. In variations where securement device 705 also includes a third suture, the third suture may have an end including a suture engagement structure in the form of a loop, disposed such that one or more of snail suture 730, boa suture 740 and suture anchor 710 pass therethrough.

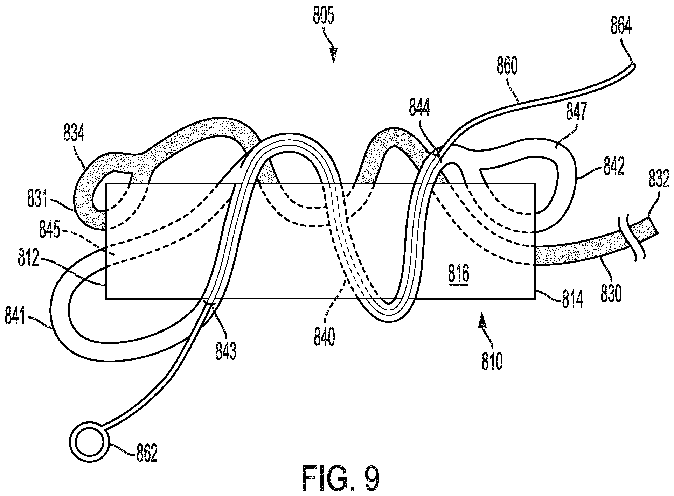

FIG. 9 depicts another embodiment of securement device, indicated by reference numeral 805. In FIG. 9, the 800 series of reference numerals refer to like elements of the 700 series of reference numerals in securement device 705 of FIG. 8, unless otherwise noted. Securement device 805 includes suture anchor 810, snail suture 830, boa suture 840 and shuttle 860. Snail suture 830 includes a length extending from a first end 831 to a second end 832. At the first end, the snail suture includes an eye splice 834 that passes through an external surface 816 of the suture anchor. From eye splice 834, snail suture 830 passes into and out of suture anchor 810, i.e., into and out of external surface 816, toward a second end 814 of suture anchor 810, as shown in FIG. 9. At second end 814, or at another location proximal to second end 814, snail suture 830 exits an opening or external surface 816 of suture anchor 810 with a remaining portion extending to second end 832, the second end being a free end. Boa suture 840 extends from first end 841 to second end 842. At first end 841 is eyelet splice 845. As shown in FIG. 9, eyelet splice 845 passes around and through first end 812 of the suture anchor, or it may pass through external surface 816, and then splices back into the body of boa suture 840 at a location closer to the other end of the suture anchor. This arrangement ensures that boa suture 840 is secured in place. Moving from first end 841 toward second end 842, boa suture 840 encircles external surface 816 of suture anchor 810 while traversing a distance of the suture anchor. Similar to first end 841, second end 842 includes eye splice 847 that passes through end 814 of suture anchor and/or external surface 816 and splices back into the body of boa suture 840. Shuttle 860 is disposed so that a portion of the shuttle passes entirely through a lumen of boa suture 840 between entry opening 843 and exit opening 844, with suture engagement structure 862 extending out of entry opening 843 and free end 864 extending out of exit opening 844.

In a variation, securement device 805 may also include a third suture with an end including a suture engagement structure in the form of a loop, disposed such that one or both of both snail suture 830 and boa suture 840 pass therethrough, or the suture engagement structure is attached directly to suture anchor. Such third suture may be adapted for use in attachment to shuttle 860 for anchorage of the securement device. In other variations of securement device 805, extreme first end 841 may be positioned within the finger trap area such that it parallels shuttle 860, and following passing of the shuttle, end 832 of snail suture 830 may pass therethrough. In some examples, an additional suture may be connected to end 832 of snail suture 830 to be passed through boa suture 840. In other variations, an additional third suture may be connected to the suture anchor and attached to the shuttle to be passed through boa suture.

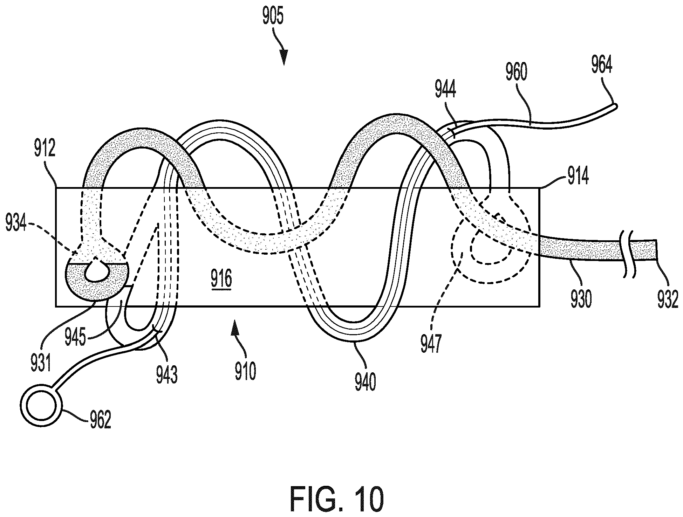

FIG. 10 depicts another embodiment of securement device, indicated by reference numeral 905. In FIG. 10, the 900 series of reference numerals refer to like elements of the 700 series of reference numerals in securement device 705 of FIG. 8, unless otherwise noted. Securement device 905 includes suture anchor 910, snail suture 930, boa suture 940 and shuttle 960. As shown in FIG. 10, securement device 905 includes eye splices similar to those included in securement device 805. One difference between securement device 905 and securement device 805 is that such eye splices are more centrally disposed in the suture anchor in securement device 905. Specifically, eye splice 934 at first end 931 of snail suture 930 is internal to end 912 of suture anchor. Such eye splice 934 may be on one side of suture anchor or may pass through suture anchor such that it is exposed on both sides. As with securement device 805, second end 932 of the snail suture is a free end. Boa suture 940 includes eye splices 945, 947 at its ends, each eye splice being internally spaced relative to respective ends 912, 914 of suture anchor 910. One or both of eye splices 945, 947 may pass through external surface 916 of suture anchor or be partially embedded in suture anchor 910 and may be disposed oriented in a depth-direction of the suture anchor. Between eye splices 945, 947, boa suture 940 extends circumferentially around a periphery and external to suture anchor 910 in a direction to traverse a distance between ends of the boa suture. Shuttle 960 is disposed so that a portion of shuttle passes entirely through a lumen of boa suture 940 between entry opening 943 and exit opening 944, with suture engagement structure 962 extending out of entry opening 943 and free end 964 extending out of exit opening 944. In a variation, securement device 905 may also include a third suture with an end including a suture engagement structure in the form of a loop disposed such that one or both of both snail suture 930 and boa suture 940 pass therethrough, or the suture engagement structure is attached directly to suture anchor.

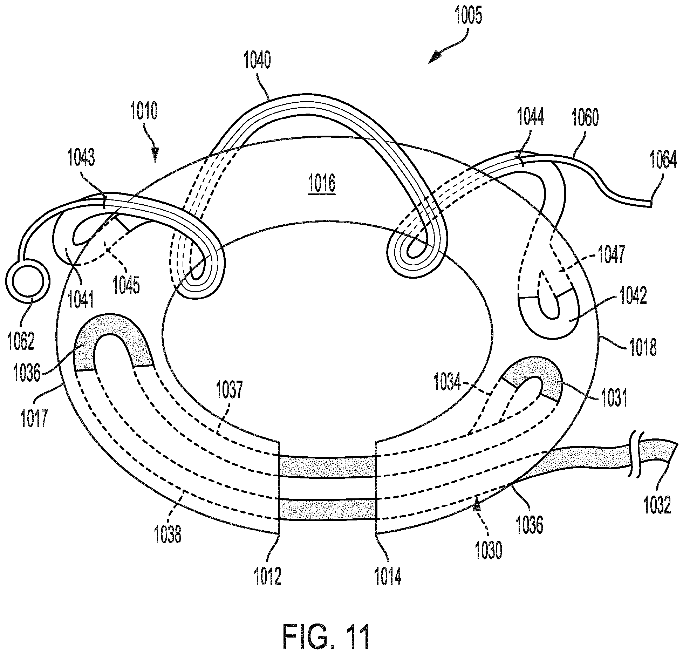

FIG. 11 depicts another embodiment of securement device, indicated by reference numeral 1005. Securement device 1005 includes suture anchor 1010, snail suture 1030, boa suture 1040, and shuttle 1060. Suture anchor 1010 may be characterized by having a "C" type shape, with first end 1012 and second end 1014 facing one another, as shown in FIG. 11. A perimeter of a body of suture anchor 1010 from end 1012 to end 1014 is enclosed aside from a portion between the facing surfaces of ends 1012, 1014, and may have a partially circular shape, an ovular shape, or any other shape to define a partial enclosure. In an assembled configuration, a maximum extent of suture anchor 1010 in one direction may be from a first outer bound 1017 and a second outer bound 1018, as indicated in FIG. 11. Snail suture 1030 extends from a first end 1031 to a second end 1032. Eye splice 1034 in part defines first end 1031, and may pass through an external surface 1016 of suture anchor twice, as shown in FIG. 11. A first segment 1037 of snail suture 1030 extends from first end 1031 proximal to outer bound 1018 to exposed portion 1036 proximal to outer bound 1017, where snail suture 1030 may pass through external surface 1016 twice to define an exposed portion. From exposed portion 1036, a second segment 1038 of snail suture 1030 laps back toward outer bound 1018 to exit location 1036, from which suture 1030 extends to free end 1032. Both first and second segments 1037, 1038 include exposed sub-portions spanning a gap between first end 1012 and second end 1014 of the suture anchor, as shown in FIG. 11.

With continued reference to FIG. 11, boa suture 1040 of securement device 1005, like snail suture 1030, also spans a distance generally corresponding to a distance between outer bounds 1017 and 1018, though not overlapping with snail suture 1030. Boa suture 1040 has a length from a first end 1041 with a first eye splice 1045 to a second end 1042 with a second eye splice 1047. Both first and second eye splices may pass through external surface 1016 or be partially embedded into suture anchor 1010. Along a distance between first and second eye splices 1045, 1047, boa suture 1040 is external to suture anchor 1010 and passes circumferentially around its perimeter while traversing a distance between the eye splices. Shuttle 1060 is disposed so that a portion of the shuttle passes entirely through a lumen of boa suture 1040 between entry opening 1043 and exit opening 1044, with suture engagement structure 1062 extending out of entry opening 1043 and free end 1064 extending out of exit opening 1044. In a variation, securement device 1005 may also include a third suture with an end including a suture engagement structure in the form of a loop, disposed such that one or both of both snail suture 1030 and boa suture 1040 pass therethrough, or the suture engagement structure is attached directly to suture anchor.

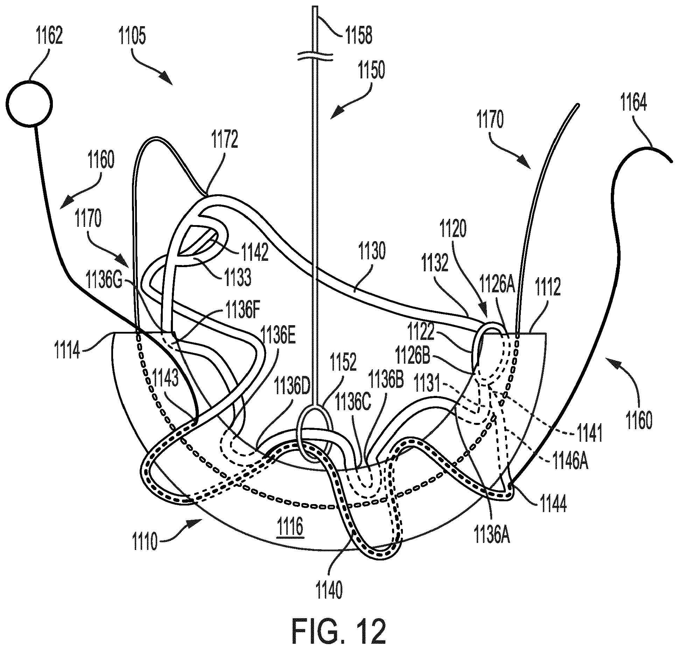

FIG. 12 depicts another embodiment of securement device, indicated by reference numeral 1105. In FIG. 12, the 1100 series of reference numerals refer to like elements of the double-digit series of reference numerals in securement device 5 of FIG. 1A, unless otherwise noted. Securement device 1105 includes a suture anchor 1110, first suture 1120, second suture 1150 and third suture 1170. Third suture 1170 passes through a lumen of suture anchor 1110 from end 1112 to end 1114, as shown in FIG. 12. In some examples, third suture 1170 passes through external surface 1116 of the suture anchor at one or more locations along a distance between ends 1112, 1114. One end 1172 may be attached to first suture 1120 so that third suture 1170 has one free end. Alternatively, both ends of third suture 1170 could be free and slidable within anchor 1110 and passed through or around tissue and secured with a traditional knot or sliding knot. In another alternative, both ends of suture 1170 could be free but the suture is not slidable within anchor 1110 (e.g., by means of being stitched to external surface 1116 or the like) such that the end(s) of suture 1170 could be passed through or around tissue and passed into another suture anchor, such as an anchor in the Omega Knotless Anchor System (Stryker Corporation, Kalamazoo, MI). The arrangement of the third suture shown in FIG. 12 may also be used in the other contemplated embodiments, including, for example, those shown in FIGs. 2A-C and 3-11.

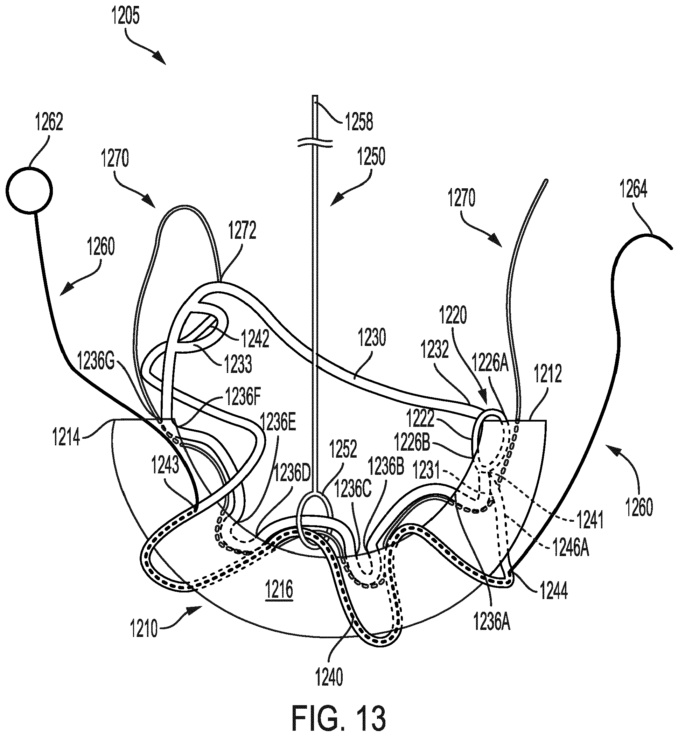

FIG. 13 depicts another embodiment of securement device, indicated by reference numeral 1205. In FIG. 13, the 1200 series of reference numerals refer to like elements of the double-digit series of reference numerals in securement device 5 of FIG. 1A, unless otherwise noted. Securement device 1205 includes a suture anchor 1210, first suture 1220, second suture 1250 and third suture 1270. Third suture 1270 is arranged so that it runs generally parallel to snail extension 1230 between ends 1212, 1214 of suture anchor 1210 as shown in FIG. 13. In some examples, third suture 1270 is directly connected to snail extension 1230 along a portion of its length. In other examples, third suture 1270 is parallel to, but offset from the snail extension. In still further examples, third suture 1270 is parallel to the snail extension over parts of a distance between ends of the suture anchor but includes some variation in offset over the distance. One end 1272 may be attached to first suture 1220 so that the third suture has one free end, or both ends of suture 1270 could be free and usable as described above as to FIG. 12. The arrangement of the third suture shown in FIG. 13 may also be used in the other contemplated embodiments, including, for example, those shown in FIGs. 2A-C and 3-11.

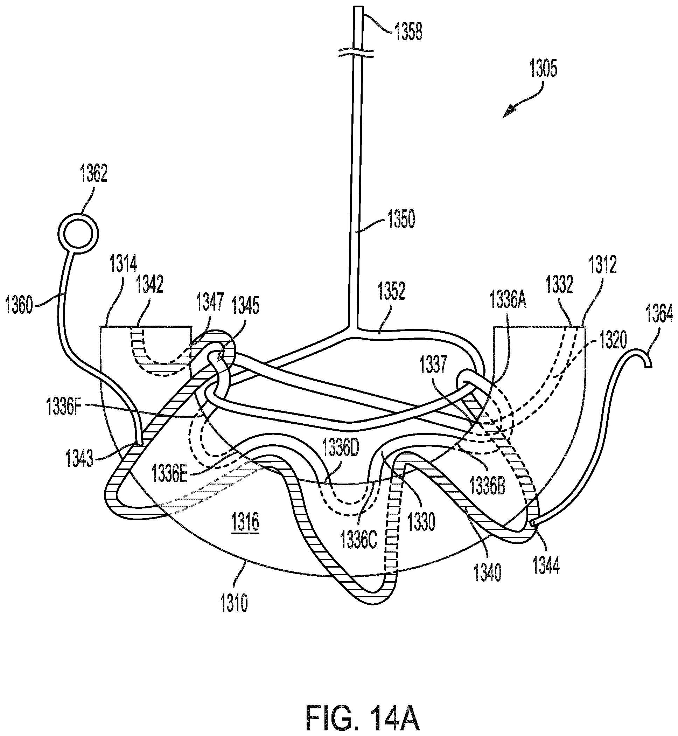

FIG. 14A depicts yet another embodiment of a securement device, indicated by reference numeral 1305. In FIG. 14A, the 1300 series of reference numerals refer to like elements of the double-digit series of reference numerals in securement device 5 of FIG. 1A, unless otherwise noted. Securement device 1305 includes a suture anchor body 1310, a first suture 1320 that includes a snail extension 1330 and a boa extension 1340, a second suture 1350 and a shuttle 1360.

Suture anchor body 1310 may have an external surface 1316 through which the first suture passes through at one or more locations on the anchor body. The first suture as depicted has a length that extends from a first end 1332 to a second end 1342, encompassing both the snail extension and the boa extension. Snail extension 1330 of the first suture extends from first end 1332 to location 1336A at a juncture with a surface of the suture anchor body. From first end 1332, snail extension passes through a lumen of body 1310 then out of the anchor body at location 1337, is spliced with the opposite end of the first suture at splice 1345, then extends into and out of suture anchor 1316 at entry and exit locations 1336A-F along the body. This arrangement constitutes the snail. From location 1336A in a direction toward second end 1342, the first suture exits the anchor body and proceeds to extend circumferentially around the anchor body toward end 1314 of the suture anchor. This circumferential wrapping of boa extension 1340 around the anchor constitutes the boa and is indicated with hatch markings in FIG. 14A. Along boa extension 1340 between location 1336A and location 1347 the suture does not penetrate the suture anchor body, though one or more penetrations by boa extension 1340 may exist if desired. While FIG. 14A illustrates the boa as having three circumferential passes around a perimeter of the anchor body, variations of the design may include any number of such circumferential passes provided that there is at least one half of a complete rotation. Splice 1345 may be a Brummel splice between a portion of the first suture near second end 1342 and a portion of the first suture near first end 1332, as shown in FIG. 14A. In variations, other types of splices or knots may be used at an intersection of the suture portions identifiable by splice 1345. It should be appreciated that in securement device 1305, a transition between snail 1330 and boa 1340 in the form of a turn of the suture occurs at a location spaced apart from end 1312 of anchor body 1310, unlike securement device 5 in FIG. 1A. In FIG. 14A, such turn is outside of the anchor body adjacent to location 1336A. In variations, the transition region may be a loop instead of a simple turn. In this manner, a portion of the boa extension and a portion of the snail extension may form part of the loop, with the loop being created via a splice or knot. In one example, such loop may be structured as described for loop 22 in FIG. 1A. As to the ends of the first suture, both ends 1332, 1342 of first suture 1320 are located generally in respective opposing end regions of anchor body 1310 without being attached or connected to other parts of the first or second sutures. Ends 1332, 1342 may be tucked into the anchor body. In at least some variations, ends 1332, 1342 are not physically attached to anchor body 10, other than via passage through external surface 1316 at locations 1337 and 1347, respectively.

Second suture 1350 includes a suture engagement structure 1352 in the form of a loop positioned to pass around boa extension 1330 outside of suture anchor 1310 and on opposite sides of a region between locations 1336A-F. Put another way, suture engagement structure 1352 passes around the snail extension adjacent to anchor end 1312 and adjacent to anchor end 1314. In this manner, second suture 1350 is operatively connected to the first suture at locations towards or at opposite ends of the suture anchor. In some examples, suture engagement structure 1352, e.g., loop of the second suture may be placed around the first suture before the first suture is fully attached to the suture anchor and before splice 1345 is created. In other examples, a splice or knot to create the loop of second suture may be created after the first suture is fully prepared with respect to the suture anchor. Shuttle 1360 passes through a lumen of first suture 1320 from opening 1343 to opening 1344, and includes leading end 1364 and suture engagement structure 1362 opposite the leading end. Second suture 1350 and shuttle 1360 may be operatively connected in the same manner as described for securement device 5. Of course, this configuration of suture engagement structure 1352 can be included in other embodiments of securement devices contemplated herein. Securement device 1305 is advantageous for at least the reasons described elsewhere in the present disclosure for securement device 5 shown in FIG. 1A.

A variation of securement device 1305 is shown in FIG. 14B in the form of securement device 1305'. In FIG. 14B, the 1300' series of reference numerals refer to like elements of the 1300 series of reference numerals in securement device 1305 of FIG. 14A, unless otherwise noted. In securement device 1305', the ends of shuttle 1360' (suture engagement structure 1362' and end 1364', respectively) each pass through a loop of suture engagement structure 1352' of second suture 1350' on opposite sides of a shuttle portion between openings 1343', 1344' on boa extension 1340' of first suture 1320'. Specifically, an end portion of shuttle 1360' including suture engagement structure 1362' extends from opening 1343' through suture engagement structure 1352', and an end portion of shuttle 1360' including end 1364' extends from opening 1344' through suture engagement structure 1352'. The shuttle arrangement in securement device 1305' provides enhanced stability and a more constrained structure once fully deployed with second suture 1350' passed into boa extension 1340'.

FIG. 20A depicts yet another embodiment of a securement device, indicated by reference numeral 1405. Securement device 1405 includes a suture anchor 1410, a suture 1430 and a shuttle 1460. Suture 1430 extends from a loop portion 1431 at a first end to a free end 1438 opposite the first end, the free end 1438 being a second end. Loop portion 1431 passes through suture anchor 1410 and into itself adj acent to location 1434A. Loop portion 1431 may be formed via a Brummel splice or a tuck splice, for example, or any other attachment means as described throughout the present disclosure. In some examples, cross-stitching may be incorporated into loop portion 1431 in a sub-portion 1432 passing through suture anchor 1410 to increase strength of the closed loop portion 1431. In terms of the attachment of suture 1430 to suture anchor 1410, starting from loop portion 1431, suture 1430 extends externally from suture anchor 1410 until reentering suture anchor at location 1434B, then continues internally through suture anchor 1410 to location 1434C. From location 1434C, suture 1430 extends out of the suture anchor 1410 and continues externally from suture anchor 1410 to location 1434D, from which location suture 1430 passes internally through suture anchor 1410 again to location 1434E. From location 1434E, suture 1430 exits suture anchor 1410 and extends to free end 1438. In this arrangement, a first external segment of suture 1430 extends from loop 1431 to location 1434B, and a second external segment of suture 1430 extends from location 1434C to location 1434D. Overall, first suture 1430 is arranged with respect to suture anchor 1410 in a snail configuration. Turning to shuttle 1460, in the pre-implantation configuration shown in FIG. 20A, shuttle 1460 is loaded into suture 1430 such that shuttle 1460 is never disposed within suture 1430 when suture 1430 is in suture anchor 1410. In the specific arrangement shown in FIG. 20A, shuttle 1460 passes through a lumen of suture 1430 between openings 1441, 1442 along the first external segment of suture 1430, then continues externally from both suture 1430 and suture anchor 1410 before it again passes through the lumen of suture 1430 between openings 1443, 1444 along the second external segment. At a first end of shuttle 1460 extending from opening 1441 is a suture engagement structure 1462 in the form of a loop, and at an opposite second end is a free end 1468.

A variation of securement device 1405 is shown in FIG. 20B in the form of securement device 1405'. In FIG. 20B, the 1400' series of reference numerals refer to like elements of the 1400 series of reference numerals in securement device 1405 of FIG. 20A, unless otherwise noted. Securement device 1405' includes suture anchor 1410', suture 1430' and shuttle 1460'. Shuttle 1460' is arranged relative to suture 1430' such that shuttle 1460' passes through a lumen of suture 1430' via openings 1441', 1442' along a first external segment of suture 1430', then exits suture 1430' and passes into suture anchor 1410' before exiting suture anchor 1410' to again pass into and through the lumen of suture 1430' via openings 1443', 1444' along a second external segment of suture 1430'.

FIG. 21A depicts yet another embodiment of a securement device, indicated by reference numeral 1505. In FIG. 21A, the 1500 series of reference numerals refer to like elements of the 1400 series of reference numerals in securement device 1405 of FIG. 20A, unless otherwise noted. Securement device 1505 includes a suture anchor 1510, a first suture 1530, a second suture 1550 and a shuttle 1560. First suture 1530 includes loop portion 1531 with sub-portion 1532 passing through suture anchor 1510. From loop portion 1531, first suture 1530 extends externally from suture anchor 1510 until reentering suture anchor 1510 at location 1534B, then continues internally through suture anchor 1510 to location 1534C. From location 1534C, first suture 1530 exits out of the suture anchor 1510 and continues externally from suture anchor 1510 to location 1534D, from which location first suture 1530 passes internally through suture anchor 1510 again to location 1534E. From location 1534E, first suture 1530 exits suture anchor 1510 and extends to free end 1538. In this arrangement, a first external segment of first suture 1530 extends from loop 1531 to location 1534B, and a second external segment of first suture 1530 extends from location 1534C to location 1534D.

In securement device 1505, second suture 1550 is connected to first suture 1530 via a splice 1581. Specifically, free end 1538 of first suture 1530 is spliced with a first end 1551 of second suture 1550. In some examples, the splice may be arranged such that a terminal end portion of first suture 1530 passes into a lumen of second suture 1550 and a terminal end portion of second suture 1550 passes into a lumen of first suture 1530. It should be appreciated that any other suitable splice may also be used. Further, in some examples, the splice may be complemented by a cross-stitch. In still further examples, the splice may be complemented by an adhesive. It should also be appreciated that in the contemplated splicing arrangement, the free end 1538 of first suture 1530 passing into the lumen of second suture 1550 at splice 1581 does not form part of a finger trap when second suture 1550 is pulled through first and second external segments of first suture 1530 via shuttle 1560. Further, in some examples, second suture 1550 may advantageously have a smaller diameter than that of first suture 1530. In such examples, passing second suture 1550 through a lumen of first suture 1530, as described in greater detail below, may be rendered easier. With continued reference to second suture 1550, the lumen of second suture 1550 in a portion of second suture 1550 proximate first end 1551 includes a core suture 1570 received therein, the core suture 1570 extending from a first end 1571 to a second end 1572 and being shorter than second suture 1550 such that the lumen in a portion of second suture 1550 proximate a second end 1558 of second suture 1550 is vacant, as shown in FIG. 21A. Core suture 1570 functions to increase the bulk of the suture such that when the implant is deployed, parts of the finger trap formed with the portion of the second suture 1550 filled by the core suture 1570 provide a tighter and stronger connection. Core suture 1570 may be fed into second suture 1550 using standard methods, such as that involving the use of a needle. In some examples, a vacant portion of second suture 1550 may have a diameter in a range from 0.10 mm to 1.30 mm while a bulked up portion of second suture 1550 with core suture 1570 disposed therein may have a diameter in a range from 0.20 mm to 2.60 mm. Second suture 1550 may also have diameters outside of this range, and such diameter may vary based on a variety of factors, such as the looseness of the braiding. In other examples, the diameter of the second suture with the core suture disposed therein may be any diameter that promotes the fixation of the second suture within the first suture.

Shuttle 1560 is loaded into first suture 1530 such that shuttle 1560 is never disposed within portions of first suture 1530 that are within suture anchor 1510. In the specific arrangement shown in FIG. 21A, shuttle 1560 passes through a lumen of first suture 1530 between openings 1541, 1542 along the first external segment of first suture 1530, then continues externally from both suture 1530 and suture anchor 1510 before again passes through the lumen of first suture 1530 between openings 1543, 1544 along the second external segment. At a first end of shuttle 1560 extending from opening 1541 is a suture engagement structure 1562 in the form of a loop, and at an opposite second end is a free end 1568.

A variation of securement device 1505 is shown in FIG. 21B in the form of securement device 1505'. In FIG. 21B, the 1500' series of reference numerals refer to like elements of the 1500 series of reference numerals in securement device 1505 of FIG. 21A, unless otherwise noted. Securement device 1505' includes suture anchor 1510', first suture 1530', second suture 1550' and shuttle 1560'. In this arrangement, first suture 1530' may attach to suture anchor 1510' in the same manner as that provided in securement device 1505, and shuttle 1560' may be loaded through first suture 1530' in the same manner as that provided in securement device 1505. However, in securement device 1505', the ends of first suture 1530' and second suture 1550' may have various configurations. As shown in FIG. 21B, free end 1538' of first suture 1530' may extend past a splice 1581' of the first and second sutures 1530', 1550', the free end 1538' exiting the lumen of second suture 1550'. In some examples, free end 1538' may reenter first suture 1530' at a location closer to or within the suture anchor 1510'. In other examples, free end 1538' may remain loose. As to second suture 1550', splice 1581' between first and second sutures 1530', 1550' is remote from both first end 1551' and second end 1558' of second suture 1550'. As second suture 1550' extends past its splice 1581' with first suture 1530', e.g., by passing into a lumen of first suture 1530', second suture 1550' exits first suture 1530' at a location on first suture 1530' that is external to suture anchor 1510'. From such location adjacent to splice 1581', second suture 1550' extends to opening 1548' and reenters the lumen of first suture 1530' between openings 1547', 1548' along the second external segment of first suture 1530', then continues externally from both first suture 1530' and suture anchor 1510' before second suture 1550' again enters the lumen of first suture 1530' via opening 1546' along the first external segment. As shown, first end 1551' of second segment 1550' terminates and is fixed within such first external segment. In some examples of this variation, the second suture may extend further, such as into the loop portion 1531'. On the other side of splice 1581', a lumen of second suture 1550' includes a core suture 1570' disposed therein in a similar manner to that provided for second suture 1550 in securement device 1505.

The securement device 1505' may be varied in many ways. In some examples, second suture 1550' may be tucked into itself after forming part of splice 1581'. In such examples, first end 1551' of second suture 1550' is located adjacent to splice 1581' and does not extend back through first suture 1530' as shown in FIG. 21B. In other examples, second suture 1550' passes through the lumen of first suture 1530' in first and second external segment as shown in FIG. 21B, but also in between, such that it extends continuously through and within the lumen of first suture 1530' from opening 1544' to a location proximate loop 1531'. In other examples, securement device 1505' may have a second suture 1550' arranged so that second suture 1550' is spliced with first suture 1530' to define splice 1581', and a portion of second suture 1550' extending to first end 1551' exits first suture 1530' and is returned into lumen of second suture 1550' to bulk up a portion of second suture 1550' with an end portion of second suture 1550'. Such an arrangement may replace the core suture with an end portion of second suture 1550'. In yet another example, second suture 1550' may be arranged with a combination of a reinserted end portion of second suture 1550' and core suture 1570' to bulk up a trailing portion of second suture 1550' remote from second end 1558'.

Further variations of securement devices 1405, 1405', 1505 and 1505' and other similar embodiments are also contemplated. In some variations, a portion of the shuttle 1460, 1460', 1560, 1560' between location 1441, 1441', 1541, 1541' and loop of suture engagement structure 1462, 1462', 1562, 1562' may be disposed to pass through suture anchor 1410. In other variations, shuttle 1460, 1460', 1560, 1560' may be arranged so that a portion between location 1444, 1444', 1544, 1544' and free end 1468, 1468', 1568, 1568' may pass into the lumen of suture 1430, 1430' or second suture 1550, 1550' for a portion of the applicable suture between location 1434E, 1434E'. 1534E, 1534E' and free end 1438, 1438', 1558, 1558'. In still further variations, the contemplated securement device may include a suture 1430, 1430', 1530, 1530' arranged so that suture 1430, 1430', 1530, 1530' has a single external segment along the suture anchor 1410, 1510 or three or more external segments. Thus, in one specific example, a securement device with a single external segment may have a suture attached to the suture anchor at opposite ends of the suture anchor, with the single external segment in between such opposite ends. In another specific example with three external segments, the suture of the securement device may pass into and out of the suture anchor at two separate locations between ends of the suture anchor.

FIG. 22A depicts yet another embodiment of a securement device, indicated by reference numeral 1605. Securement device 1605 includes suture anchor 1610, first suture 1630, second suture 1650 and shuttle 1660. First suture 1630 extends from a first end to a second end where the first end includes loop portion 1631 and the second end includes loop portion 1636. Between the first and second ends, first suture 1630 includes two external segments, both external to suture anchor 1610. The first external segment extends from location 1634A to location 1634B, while the second external segment extends from location 1634C to location 1634D, as shown in FIG. 22A. In between locations 1634B and 1634C, first suture 1630 passes into suture anchor 1610. Additionally, each loop portion 1631, 1636 is attached to suture anchor 1610 at respective ends of suture anchor 1610. As with other embodiments of the present disclosure, each loop portion 1631, 1636 may be formed through a splice or other means as contemplated by the present disclosure.

Second suture 1650 includes a first end with a loop portion 1651 that is attached to first suture 1630 by passing loop portion 1651 through loop portion 1636. Such attachment may be formed by splicing the respective loops once the suture of each is in position, or by splicing one loop portion after the other loop portion. A second end of second suture 1650 is a free end 1658 and is opposite loop portion 1651. Shuttle 1660 has a length extending from suture engagement structure 1662 to a free end 1668, where suture engagement structure 1662 includes a closed loop of suture. In the specific arrangement shown in FIG. 22A, shuttle 1660 passes through and within a lumen of first suture 1630 between openings 1641, 1642 along the first external segment of first suture 1630, then continues externally from both first suture 1630 and suture anchor 1610 before shuttle 1660 again passes through and within the lumen of suture 1630 between openings 1643, 1644 along the second external segment. In an alternative arrangement of securement device 1605, first suture 1630 may be attached to suture anchor 1610 only at loop portion 1631 and between locations 1634B, 1634C, so that loop portion 1636 is free floating. Such an arrangement may provide a more robust and streamlined finger trap when second suture 1650 is passed through first suture 1630 via shuttle 1660, although may produce a final implant where the loop portions 1636, 1651 themselves form part of the finger trap.

A variation of securement device 1605 is shown in FIG. 22B in the form of securement device 1605'. In FIG. 22B, the 1600' series of reference numerals refer to like elements of the 1600 series of reference numerals in securement device 1605 of FIG. 22A, unless otherwise noted. Securement device 1605' includes suture anchor 1610', first suture 1630', second suture 1650' and shuttle 1660'. In this arrangement, first suture 1630' is a continuous closed loop, as shown in FIG. 22B. The closed loop of first suture 1630' may be formed through attachment of suture ends via a splice or other means as contemplated by the present disclosure. As depicted, first suture 1630' passes through suture anchor 1610', i.e., attaches to suture anchor 1610', in three separate regions: between locations 1634A' and 1634B', between locations 1634C' and 1634D' and between locations 1634E' and 1634F'. Outside of these regions, first suture 1630' is external to suture anchor 1610'. Second suture 1650' includes a loop portion 1651' at a first end and a free end 1658' opposite the loop portion 1651'. Loop portion 1651' is arranged to couple to first suture 1630' with first suture 1630' passing through loop portion 1651', and may do so at one end of suture anchor 1610', as shown in FIG. 22B. Shuttle 1660' passes through a lumen of first suture 1630' via and between openings 1641', 1642' along an external segment of first suture 1630'. Otherwise, shuttle 1660' may be external to a remainder of securement device 1605' such that ends 1662', 1668' are free ends. In an alternative example of this variation, first suture 1630' may pass through and attach to suture anchor 1610' to a different extent in the vicinity of locations 1634A' and 1634B' than that shown in FIG. 22B. In still further alternative examples, first suture 1630' may attach to suture anchor 1610' at two separate locations in the vicinity of location 1634A' such that a portion of first suture 1630' in between such entry locations defines a short loop external to and on the left side of suture anchor 1610' in FIG. 22B.

FIG. 23A depicts yet another embodiment of a securement device, indicated by reference numeral 1705. Securement device 1705 includes suture anchor 1710, first suture 1730 and shuttle 1760. In this arrangement, suture anchor 1710 is a hard anchor. Examples of such hard anchor include the NanoTack^{®} anchor by Stryker^{®} and the anchors described in U.S. Pat. No. 10,426,456 ("the '456 Patent"), the entire disclosure of which is incorporated by reference herein. Suture anchor 1710 as depicted is an expandable anchor with an outer surface having a series of spaced apart ribs 1719 and a channel 1714 extending longitudinally therethrough, the channel 1714 being accessible at least from a trailing end 1711 of the suture anchor 1710. Along a length of the channel is a step 1715 separating a narrower portion of the channel 1714 proximate the trailing end 1711 from a wider portion of the channel 1714 proximate a leading end 1712 of the suture anchor 1710. Disposed within the wider portion of the suture anchor 1710 is an optional slidable deployment cylinder 1720, also cannulated. First suture 1730 includes an enlarged first end 1731 disposed adjacent to or within the wider portion of channel 1714, a remainder of first suture 1730 extending through the channel 1714 and out of trailing end 1711 of suture anchor 1710. A lumen through a portion 1734 of first suture 1730 is arranged proximate trailing end 1711 prior to deployment and receives shuttle 1760 therethrough, as shown in FIG. 23A. Shuttle 1760 is received through the lumen in portion 1734 such that a suture engagement structure 1762 of the shuttle 1760 with a loop portion extends from an opening at one end of the portion 1734 and a free end 1768 of shuttle 1760 extends from an opening at an opposite end of the portion 1734. The shuttle may be arranged such that an alignment of the shuttle within the lumen of the first suture 1730 corresponds to the part of the shuttle extending to free end 1768, as shown in FIG. 23A, with the suture engagement structure end being bent backward to be directed away from the suture anchor 1710, as shown in FIG. 23A. In an alternative variation of securement device 1705, suture anchor 1710 may be replaced with a button or other similar device as known to persons of ordinary skill in the art. Greater detail on the operation and deployment of the securement device 1705 is provided in the description of its method of use elsewhere in the present disclosure.

In some embodiments, an anchorage arrangement may include two or more securement devices. One example of such arrangement is shown in FIG. 15A, where first and second securement devices 2105, 2205 are disposed in respective bone holes 2102, 2202 in a bone 2101. As depicted in FIG. 15A, each securement device includes components that are the same as those included in securement device 5. When secured into place with second suture 2150 passed through soft tissue 2103 and then attached to shuttle 2160, a leading end of shuttle 2160 may be passed through tissue 2103 twice, as shown in FIG. 15A, and then attached to suture engagement structure 2262 of shuttle 2260 in the second securement device 2205. This creates a "daisy chain" and a "suture bridge" utilizing shuttle 2160, bringing the soft tissue toward the bone surface. And, through this step, second suture 2150 may be pulled through at least the boa suture in securement device 2105 while shuttle 2160 takes on an operative function by being pulled into the boa suture of securement device 2205 as leading end 2264 of shuttle 2260 is pulled. Through this configuration, a portion of the first securement device 2105 may be used to create a one-way tensioning mechanism in second securement device 2205. In some variations of this example, second suture 2250 may be passed through soft tissue 2103 and then secured to shuttle 2260 at end 2262. The approach shown in FIG. 15A may be similarly employed using the other securement devices contemplated by the present disclosure.

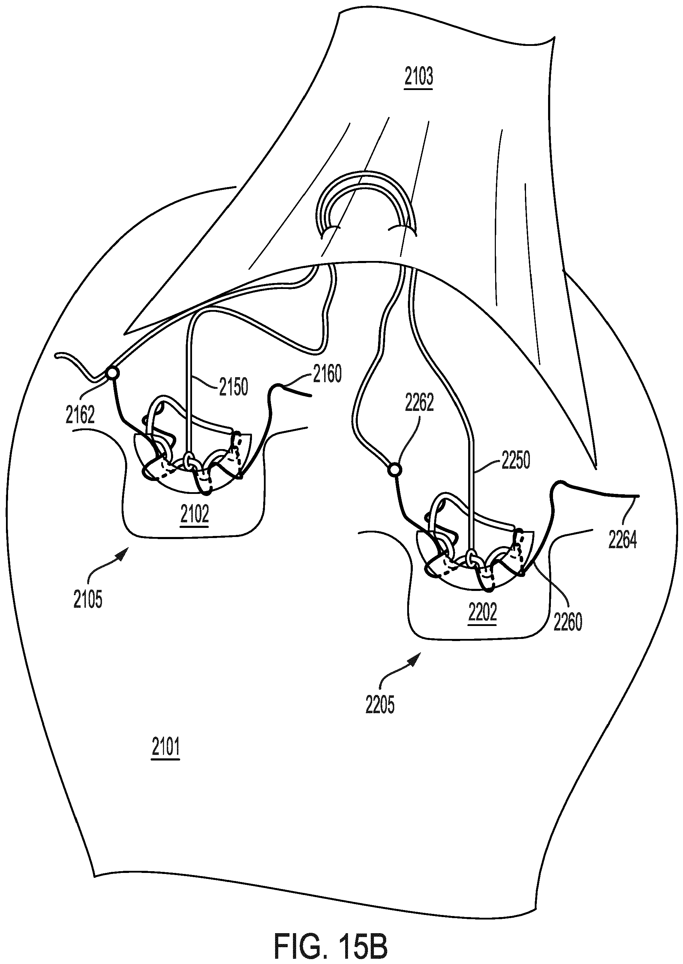

FIG. 15B illustrates an alternative way to connect two securement devices. In this example, the second suture 2150 of first securement device 2105 is passed through tissue 2103 and then inserted into suture engagement feature 2262 of second securement device 2205. And similarly, the second suture 2250 of second securement device 2205 is passed through tissue 2103 and then inserted into suture engagement feature 2162 of first securement device 2105.

The securement device may be varied in many ways. In variations of securement device 5, 105, 105', 105", 205, 305, 405, 505, 605, 705, 805, 905, 1005, 1105, 1205, 1305, 1305', 1505, 1505', 1605, 1605'the first suture may be cylindrical in section and the second suture may be flat in section.

In some embodiments, the suture engagement structure of the second suture, and/or third suture, as applicable, provided in securement devices 5, 105, 105', 105", 205, 305, 405, 505, 605, 705, 805, 905, 1005, 1105, 1205, 1305, 1305', 1605, 1605' may comprise an eyelet pair including two adjacent eye splices. In some embodiments, a chain-link may be formed using an end of a second suture and an end of a third suture. The respective loops of the eyelet pair or chain-link may each have a separate suture portion pass therethrough. For example, in a variation of securement device 5, boa extension 40 may pass through one eyelet and snail extension 30 may pass through another eyelet. In other examples, a chain-link may incorporate loop portion 22 such that one eyelet derives from a suture engagement structure of the second suture and the other is loop portion 22 of the first suture. Although the above examples describe specific arrangements of eyelet pairs and chain-links, it should be appreciated that such examples are merely illustrative and eyelet pairs and chain-links may be formed in other ways on the securement device. For instance, the eyelet defining the suture engagement structure of the second suture may be modified to define an eyelet pair for any variation of the securement device contemplated in the present disclosure. In still further examples, an end portion of the second suture may be coupled to an end portion of the first suture by an opposing end-to-end tuck splice. Similarly, in some other examples, an end portion of the second suture may be coupled to an end portion of the first suture by an opposing finger trap structure.

In some embodiments, a securement device may include a first suture as provided for in FIGs. 1A and 2-13, but with a modification to the snail extension such that it passes entirely through a lumen of the suture anchor from one end to the other. In such cases, the suture anchor may be modified to have characteristics including spaced apart openings along an inner side so that the snail extension is exposed in these openings. One example of such an arrangement of sutures is the IntelliBraid Technology by Stryker^{®}.

In some embodiments, the suture engagement structure of the second suture may be provided by a configuration other than eye splices such as tuck splices and brummel splices. In some examples, the suture engagement structure may be a braided loop or a knotted loop. In further embodiments, the second suture may be spliced directly to the snail suture, the boa suture, or both. In still further embodiments, the second suture may be knotted to the snail suture, the boa suture, the suture anchor, or any combination of the three.

In some embodiments, the securement device may include one or more sutures that have at least one indicating marker. These may be included on the snail suture or the boa suture, for example. The indicating marker may indicate to an operator, such as a surgeon, whether or not the securement device is properly positioned and/or compressed. The indicating marker may be, for example, a spot, a radial ring, a portion having a differing color from the rest of the suture, or the like. In another example, the indicating markers of the suture may be a portion of the suture being of a different color than the rest of the suture. Contrasting colors of the portions may provide a clear indication to the operator when performing a surgical procedure, and may be of particular use in arthroscopic procedures.

Further, in some of the aforementioned embodiments, the suture or sutures of the securement device may also include a color or other pattern along its length. For example, the suture may be of a certain color such that an operator may know which suture, among numerous others which may be present at the surgical site, is the is the suture desired to be manipulated or evaluated. Moreover, the suture may have multiple colors or patterns along its length, other than those represented as the at least one indicating marker. For example, one half of the suture may be one color, and the other half of the suture may be a different color. If the particular surgery requires that the two ends of the suture be tied together, such as by a slip knot, the two different colors may assist the operator in knowing which half of the filament should be used as the post and which half should be used to tie the slip knot around the post. Such a decision would be based on the particular surgical procedure being performed. Of course, the two differing colors may cover different amounts of the length of the suture, however, the differing colors may be most useful if they cover, where applicable, at least two free ends or end portions of the suture such that an operator can easily differentiate between the two lengths of the suture.

In still further embodiments, the soft tissue securement device may be arranged in a configuration where the shuttle is passed entirely through and out of a suture in which it is initially disposed. In one such example, securement device 5 of FIG. 1A may include suture anchor 10, first suture 20, and second suture 50 partially disposed through first suture between openings 43, 44 on first suture 20 such that a trailing portion of second suture 50 between suture engagement structure 52 and opening 43 defines an adjustable loop, and leading end 58 of second suture extends out of opening 44. It should be appreciated that in this example, shuttle 60 is not part of or is no longer part of the apparatus. Securement device 5 may be provided to a user in such configuration. The loop defined by the second suture may then be used to pass soft tissue through the loop such that when end 58 of second suture is pulled, the soft tissue is pulled into the deployed anchor. In yet another example, securement device 1305' may have a similar arrangement to that of the immediately preceding example, as shown in FIG. 14B'. In FIG. 14B', securement device 1305' of FIG. 14B is shown with shuttle 1360' passed through and removed from the boa extension 1340' of first suture 1320', leaving an adjustable loop based on a trailing portion of second suture 1350'. Soft tissue 1303' may be passed through such loop as shown in FIG. 14B' prior to completing the deployment of securement device 1305' by pulling on end 1358' of second suture 1350'. Further, a similar arrangement is also shown in FIG. 23B', where securement device 1705 includes suture anchor 1710 and suture 1730, where suture 1730 has an adjustable loop to receive soft tissue 1703, e.g., tendon, passing therethrough. Additionally, it should be appreciated that the securement devices 405, 505, 605, 705, 805, 905, 1005, 1105, 1205, 1305, 2105, 2205, 1405, 1405', 1505, 1505', 1605, 1605' depicted in FIGs. 5-13, 14A, 15A-B, 20A-B, 21A-B and 22A-B, respectively, may also be arranged in a manner as described for the above examples, where a shuttle is entirely passed through a suture and removed, leaving an adjustable loop for passage of soft tissue.

The principles illustrated by the above examples may be applied to embodiments with two separate finger traps, each with a separate suture end to control adjustment of an adjustable loop of the securement device. In one example, securement device 105 shown in FIG. 2A may include suture anchor 110, first suture 120, second suture 150 and third suture 170. Second suture 150 may be disposed through boa extension 140, while third suture 170 may be disposed through snail extension 130, with ends of the respective second and third sutures extending out of the respective extensions of the first suture. Second suture 150 and third suture 170 each define an adjustable loop. In a manner similar to that described for the above embodiments, shuttles 160, 180 are not part of or are no longer part of the apparatus in this configuration. In operation, a soft tissue may pass through a first loop created by second suture 150, a second loop created by third suture 170, and then pass back through the first loop of second suture 150 again. This last step may be performed after cinching the second loop, which may then be followed by cinching of the first loop. It should be appreciated that the securement devices depicted in FIGs. 2B-2C or other embodiments with two or more finger traps formed with separate leading ends may be similarly arranged.

In another aspect, the present disclosure relates to a kit including two or more securement devices. In one example, a kit may include two or more of a single type of securement device. In another example, a kit may include one or more of a first type of securement device and one or more of a second type of securement device. In yet another example, a kit may include a plurality of securement devices encompassing three or more types of securement devices. Any combination of securement devices may also be included in a single package or in individual packages which may later be brought together to create a kit. It is also contemplated that a kit may include any combination of securement devices along with one or more instruments used to place such securement devices in a patient, such as an anchor needle or drilling tool. In other examples, the kits contemplated herein may be accompanied by an instruction manual on how to perform one or more of the methods of using the contents of the kit.

In another aspect, the present disclosure relates to a method of fabrication of a securement device. In some embodiments, the securement devices shown in FIGs. 1A-14B and 20A-22B may be fabricated inclusive of the suture anchor with one or more of the sutures partially integrated therein or passing therethrough. For instance, securement device 5 may be fabricated such that loop portion 22 is partially embedded in suture anchor 10 and parts of snail extension 30 and boa extension 40 extending from loop portion 22 are also embedded in suture anchor 10. The fabrication of the suture anchor inclusive of one or more of the associated sutures allows for the various loops, eyelets, suture passages, connections, splices and other features of the contemplated embodiments to be more easily realized or in some instances facilitates fabrication. In some embodiments, any portion of the working sutures may be braided onto the suture anchor during manufacture. For example, the boa suture may be braided onto the suture anchor, thereby creating an at least partially integrated structure.

In another aspect, the present disclosure relates to methods of securing soft tissue using one or more securement devices to complete a repair. The securement devices of the present application may be employed in many locations in the human or other mammalian body. Exemplary, yet non-limiting applications include the rotator cuff and the labrum of the hip and the shoulder.

In one embodiment, a method involves preparing and placing one or more securement devices as contemplated by the present disclosure in a labrum of a patient. In one example, such method may involve the implantation of securement device 5, as shown in FIGs. 16-19. While FIGs. 16-19 illustrate the implantation of a single securement device 5, it should be understood that any number of securement devices may be implanted, as circumstances may deem appropriate. In a first step 2001 shown in FIG. 16, a drill 91 is used to prepare a hole 2 in a bone 1, e.g., in a glenoid, the hole being prepared for receipt of the securement device. The location of the hole in the bone may be determined based on a location of soft tissue to be repaired and/or the nature of the repair sought to be performed. The drill used may be any drill suitable to prepare a hole for a suture anchor, such as the Iconix^{®} disposable drill by Stryker^{®}.

In a second step 2002 shown in FIG. 17, a suture anchor inserter 92 is used to deliver securement device 5 into the prepared bone hole 2. In some examples, securement device 5 is fully assembled prior to the commencement of surgery such that suture anchor 10, first suture 20 and second suture 50 are attached to one another while shuttle 60 may be disposed through a lumen in first suture 20, as shown in FIG. 1A, for example. Once suture anchor 10 is advanced to a desired depth in the bone hole, third step 2003 may be performed. Free end 58 of second suture is retrieved and brought through a soft tissue 3, e.g., labrum tissue, that is sought to be secured using anchor 10, as shown in FIG. 18 for example. Once free end 58 is satisfactorily through the soft tissue, it is brought back to suture engagement structure 62 of the shuttle and secured thereto using a knot, a splice, a tuck, or other means known to those of ordinary skill. In a fourth step 2004, shown in FIG. 19, free end 64 of shuttle is now retrieved and pulled. This brings free end 58 of second suture 50 through the lumen of boa extension 40 in first suture 20 such that part of or all of shuttle 60 is drawn through the passage in the boa extension. In some examples, free end 58 of second suture is drawn until it is exposed out of exit opening 44. While second suture 50 is pulled through the lumen in boa extension 40, a one-way tensioning mechanism, also referred to herein as a finger trap, is created within the lumen, thereby locking the second suture while simultaneously strengthening the securement of suture anchor 10 within bone hole 2.

Further elaboration and examples respecting the one-way tensioning mechanism are described through the following. The one-way tensioning mechanism may be a portion of the length of suture, such as through the boa extension of the first suture, which is hollow and able to receive a free end of a suture therethrough. As already contemplated by the present disclosure, a typical suture for use in orthopedic applications may have a hollow, cylindrical shape. Entry into a hollow interior is possible by passing through the braided suture construct, i.e., in between the braided fibers. Alternatively, the braided fibers can be cut to form a hole through a braid construct and thus access into a hollow interior or the braid construct may be fabricated with prepared holes for access. A finger trap configuration can be formed in a variety of ways. In the embodiments within the present disclosure, a one-way tensioning mechanism may include a shuttle passing through the portion of suture capable of engaging the particular free end of suture and pulling it through the portion of suture, forming the finger trap construct. One example of this is found in securement device 5, where second suture 50 passes through a portion of boa extension 40 between entry opening 43 and exit opening 44. Typically, the shuttle is a length of suture or Nitinol wire, but can be formed of other materials as desired. As described above, passing or shuttling at least a portion of the length of suture through a one-way tensioning mechanism may be performed in the patient, or outside the patient wherein lengths of suture extend out of the patient through a surgical cannula.

In other examples, the method of anchoring soft tissue may include anchoring two or more securement devices. In further examples, the method may be performed as described above and shown in FIGs. 16-19 using one or more securement devices 205, 305, 405, 505, 605, 1305, 1305' shown in FIGs. 3-7, 14A and 14B, respectively. In still further examples, methods may use a securement device 5, 205, 305, 405, 505, 605, 1305, 1305' modified so that a portion of shuttle 60 is exposed out of boa extension 40 in a segment of boa extension 40 that wraps around suture anchor 10, and second suture 50 may be operatively connected to this exposed portion of shuttle 60. In these arrangements, the method may be performed as described above and shown in FIGs. 16-19. However, when shuttle end 64 is pulled as described in steps 2003 and 2004, a leading end 58 of suture 50 connected to the shuttle 60 (and/or end(s) of other suture(s) connected to the shuttle) eventually gets pulled through suture engagement structure 52 of the same suture, since it is only shuttle 60 that is connected to structure 52. In this manner, once the anchor is fully deployed, second suture 50 forms part of a one-way tensioning mechanism in the boa extension, while also having a closed-loop end passing through soft tissue 3. These principles may also be applied with the use of any one of securement devices 105, 105', 105", 1105, 1205.

In another embodiment, the method may be performed using securement device 105 of FIG. 2A. In examples of such embodiment, initial steps may be performed in a manner similar to steps 2001 and 2002 in FIGs. 18 and 19, respectively. Upon reaching a third step, while second suture 150 may be similarly passed through a first soft tissue as was described for step 2003, in this embodiment, third suture 170 is also passed through a second soft tissue different from the first soft tissue, or at a second location on the first soft tissue different from where the second suture 150 was passed. And, while second suture 150 passes through the first soft tissue to attach to securement structure 162, third suture 170 passes through the second soft tissue or the second location on the first tissue to attach to suture engagement structure 182. And, in a fourth step to anchor the suture anchor, second suture 150 is pulled via end 164 of first shuttle 160 while third suture 170 is pulled via end 184 of second shuttle 180. Upon completion, two tissues (or two portions of a single tissue) may be anchored by the single suture anchor. Securement device 105' shown in FIG. 2B may be utilized in a method of tissue anchorage in the same manner as that described above for securement device 105.

In another embodiment, securement device 105" shown in FIG. 2C may be used to perform the method. Once securement device 105" is in place in a bone hole, second suture 150", or in some cases a third suture 170" when included as part of the securement device, may be passed through a first tissue location of a tissue to be secured and then an end 158" of second suture 150" may be secured to suture engagement structure 162". Then, a first one-way tensioning mechanism may be created by pulling second shuttle 180" from a portion exposed from opening 188" to bring second suture 150" through snail extension 130". At this juncture, the first soft tissue location is secured to the anchor. Optionally, second shuttle 180", now passed entirely through snail extension 130", may be cut from second suture 150". This may be through a cut at suture engagement structure 182". Then, a now free end of second shuttle 180" may be passed through a second tissue location and then attached to suture engagement structure 162". A second one-way tensioning mechanism may be created through pulling of first shuttle 160" with second shuttle 180" attached, the second shuttle passing into boa extension 140" thereby functioning as a working suture. In some examples, a free end of second shuttle 180" may be used to anchor to a separate suture. In further examples, third suture 170" may be used and passed through any location on soft tissue, then attached to first shuttle at suture engagement structure 162" or at another location on securement device 105".

Certain repairs may be amenable to use of one or more of securement devices 105, 105', 105", while in other cases, the securement device of FIG. 1A may be used in more than one location instead of the securement devices of FIG. 2A-C to achieve a desired result.

In another embodiment, the method may be performed using securement device 1105, 1205 as shown in FIGs. 12 and 13, respectively. In such methods, steps 2001 and 2002 may be performed as described for securement device 5. In some examples, once the suture anchor is positioned in a bone hole, a free end of suture 1170, 1270, may be attached to suture engagement structure 1162, 1262 so that both second and third sutures are attached to shuttle 1160. In this manner, both sutures form part of the one-way tensioning mechanism created after the second and third sutures are passed through soft tissue in accordance with step 2003. The second and third sutures may be passed through nearby or remote locations on a soft tissue in these methods, or may pass through separate tissues. In other examples, a second shuttle may be added through at least part of snail extension 1130, 1230 so that the second and third sutures may be passed through separate suture extensions to create two one-way tensioning mechanisms.

In another embodiment of the method, the method may be performed using securement device 705 shown in FIG. 8. In one example, a bone hole is prepared as described in step 2001 and shown in FIG. 15A and the securement device 705 is inserted into the hole as described in step 2002 and shown in FIG. 17. When the securement device is positioned at an appropriate depth within the bone hole, end 732 of snail suture 730 is retrieved and brought through the tissue to be repaired. Once it is passed through such tissue, it is secured to suture engagement structure 762. At this juncture, the securement device may be locked into place by pulling end 764 of shuttle, bringing a portion of snail suture 730 through a lumen of boa suture 740, thereby creating a one-way tensioning mechanism.

In yet another example, securement device 705 may include a third suture anchored to snail suture 730, boa suture 740 and/or anchor 710, where anchorage of the third suture may be at any location along a length of suture anchor 710. In a method using such a modified securement device, the third suture may be passed through a second tissue (or a second portion of the first tissue) separate from the first portion of a first tissue passed through via snail suture 730. In some variations, the third suture, once passed through a second location, may be anchored to loop 734. In other variations, the third suture, once passed through the second location, may be attached to suture engagement structure 762 so that when end 764 of shuttle 760 is pulled, both snail suture 730 and the third suture are brought through the lumen of boa suture 740 to create a finger trap. In still further examples, securement device 705 may be modified to include a third suture as already described along with a second shuttle. In such configuration, the second shuttle may be disposed through a segment of snail suture 730 or through several portions of snail suture 730 external to suture anchor 710. In the method of securing such anchor, the third suture may pass through a second location on soft tissue and then be attached to a suture engagement structure of the second shuttle, such as an eyelet, so that pulling the second suture through the snail suture via the second shuttle creates a second finger trap to complement the first finger trap in the boa suture. Through the inclusion of a third suture in the various examples described above, a repair of soft tissue may be enhanced.

Securement devices 805, 905 and 1005 shown in FIGs. 9, 10 and 11, respectively may be used in a method of anchoring soft tissue similar to the approaches described for securement device 705 and variations thereof.

In another embodiment of the method of soft tissue repair, the method may be performed using securement device 1405 shown in FIG. 20A or securement device 1405' shown in FIG. 20B. In a first step, a drill may be used to prepare a hole in a bone, e.g., a glenoid, such that the hole is prepared for receipt of securement device 1405. This method step may be the same as that shown in FIG. 16. The location of the hole in the bone may be determined based on a location of soft tissue to be repaired and/or the nature of the repair sought to be performed. The drill used may be any drill suitable to prepare a hole for a suture anchor, such as the Iconix^{®} disposable drill by Stryker^{®}.

In a second step, a suture anchor inserter is used to deliver securement device 1405 into the prepared bone hole. In some examples, securement device 1405 is fully assembled prior to the commencement of surgery such that suture anchor 1410 and suture 1430 are attached to one another while shuttle 1460 may be disposed through a lumen in suture 1430, as shown in FIG. 20A, for example. Once suture anchor 1410 is advanced to a desired depth in the bone hole, a third step may be performed. Free end 1438 of suture 1430 is retrieved and brought through a soft tissue, e.g., labrum tissue, that is sought to be secured using anchor 1410. Once free end 1438 is satisfactorily passed through the soft tissue, it is brought back to suture engagement structure 1462 of the shuttle and secured thereto by inserting the free end 1438 through the loop of suture engagement structure 1462 and folding the free end 1438 over the loop of suture engagement structure 1462. In some examples, additional creasing of the folded free end 1438 may be performed to enhance the coupling. In still further examples, the free end 1438 may be coupled to suture engagement structure 1462 through a tuck. In a fourth step, free end 1468 of shuttle 1460 is retrieved and pulled. This brings free end 1438 of suture 1430 through the lumen of suture 1430 in the first external segment (between openings 1441 and 1442) and second external segment (between openings 1443 and 1444) such that part of or all of shuttle 1460 is drawn through such external segments. In some examples, free end 1438 of suture 1430 is drawn until it is exposed out of opening 1444. While a portion of suture 1430 adjacent to free end 1438 is pulled through the lumen in suture 1430, a one-way tensioning mechanism, i.e., finger trap, is created within the lumen, thereby locking the suture 1430 while simultaneously strengthening the securement of suture anchor 1410 within the bone hole.

A method of soft tissue repair with deployment of securement device 1405' may be performed in the same way as that described for securement device 1405. Further, securement devices 1505, 1505', 1605, 1605' may also be deployed in a similar manner to that that described for securement device 1405, where the second suture 1550, 1550', 1650, 1650' is passed through soft tissue and attached to suture engagement structure 1562, 1562', 1662, 1662' of the shuttle. With particular reference to securement devices 1505, 1505', second suture 1550, 1550' includes a core suture 1570, 1570' disposed therein, a length of core suture 1570, 1570' being shorter than that of second suture 1550, 1550'. Specifically, second suture 1550, 1550' and core suture 1570, 1570' are arranged such that a portion of second suture 1550, 1550' has a hollow lumen portion between a second end 1572, 1572' of core suture 1570, 1570' and second end 1558, 1558' of second suture 1550, 1550'. Such hollow lumen portion is long enough so that when the second suture 1550, 1550' is affixed to the shuttle 1560, 1560' and is passed through the lumen of first suture 1530, 1530', the hollow lumen portion passes through the lumen in the first and second external segments before a portion of the second suture 1550, 1550' that includes the core suture 1570, 1570' is passed through the same external segments. This arrangement ensures that the second suture 1550, 1550' does not encounter excessive resistance passing through the lumen of the first suture 1530, 1530' before the bulkier part of the second suture 1550, 1550' inclusive of the core suture 1570, 1570' is passed through the lumen of the first suture 1530, 1530'. Further, a benefit of the inclusion of core suture 1570, 1570' is realized through the creation of a tighter finger trap formed through the combination of the portion of the second suture 1550, 1550' with the core suture 1570, 1570' and the first and second external segments of first suture 1530, 1530'.

In yet another embodiment, a method of securing soft tissue using one or more securement devices involves use of securement device 1705, shown in FIGs. 23A-C. The method commences with preparation of securement device 1705 components and also preparation of a bone surface in a joint, such as a shoulder or hip, to receive the securement device 1705. Such method steps may include one or more of the method steps described in the '456 Patent.

In one example, the method may be performed using a suture anchor system including securement device 1705, an inserter, a guide and a drill. This method is described below as being applied to the glenoid and labrum in the shoulder, though it should be appreciated that it could be employed in other joints, such as the hip. An inserter may include a hollow push tube having a lumen extending therethrough. The inserter terminates at its distal end in a drive surface for engaging the proximal end of suture anchor 1710, and terminates at its proximal end in a handle. The handle may include features to secure the free ends of suture 1730 and shuttle 1760, e.g., one or more suture cleats, suture slots, suture clamps, etc. Where such features are provided, and where appropriate, handle may also include one or more release mechanisms to release the free ends of suture 1730 and shuttle 1760. Handle may also have one or more mechanisms to apply tension to one or more free ends of suture 1730 and shuttle 1760. Suture 1730 extends through lumen of hollow push tube. By maintaining a slight proximally-directed tension on the proximal end of suture 1730, suture anchor 1710 can be held against the drive surface of hollow push tube, thereby providing a degree of control for maneuvering the suture anchor 1710.

Preferably suture anchor 1710, suture 1730 and the inserter are pre-assembled into a single unit, with suture 1730 extending back through the lumen of the inserter with a slight proximal tension so as to hold suture anchor 1710 on the distal end of the inserter. Shuttle 1760 may also be part of the pre-assembled single unit.

The suture anchor system may also include a hollow guide for guiding components from outside of the body to the glenoid. More particularly, the hollow guide generally comprises a lumen for slidably receiving suture anchor 1710 and the inserter therein. The internal diameter of the hollow guide is preferably approximately equal to the largest external feature of suture anchor 1710, so that suture anchor 1710 can make a close sliding fit within the interior of the hollow guide. Alternatively, the internal diameter of the hollow guide may be slightly smaller or larger than the largest external feature of suture anchor 1710 if desired. Where the suture anchor system also comprises a punch or drill, the lumen of the hollow guide is preferably sized to slidably receive the punch or drill. The distal end of the hollow guide preferably includes a sharp tip/edge for penetrating the labrum and engaging the glenoid.

If desired, the suture anchor system may also include a punch or drill having a sharp distal end and a proximal end having a handle mounted thereto. The handle may include a mount for the drill so as to facilitate turning the drill with a powered driver, etc. Again, the sharp distal end of the punch or drill is adapted to penetrate the glenoid.

The suture anchor system is preferably used as follows to secure a detached labrum to the glenoid.

First, the sharp distal end of the hollow guide is passed through the labrum and positioned against the glenoid at the location where suture anchor 1710 is to be deployed. Preferably the sharp distal end of the hollow guide penetrates through the labrum and a short distance into the glenoid so as to stabilize the hollow guide vis-à-vis the glenoid. A stylet (e.g., an obturator) may be used to fill the hollow guide during such insertion and thus prevent tissue coring of the labrum during insertion. The distal portion of the punch or drill may also be used to fill the hollow tip of the hollow guide during such insertion.

Next, if desired, the punch or drill may be used to prepare a seat in the glenoid to receive suture anchor 1710. More particularly, if the punch or drill is used, the sharp distal end of the punch or drill is passed through the hollow guide (thereby also passing through the labrum) and advanced into the glenoid so as to form an opening (i.e., a seat) in the bone to receive suture anchor 1710. Then, while the hollow guide remains stationary, the punch or drill is removed from the hollow guide.

Next, the inserter, carrying suture anchor 1710 thereon, is passed through the hollow guide (thereby also passing through the labrum) and into the seat formed in the glenoid. As suture anchor 1710 is advanced into the bone, the body of suture anchor 1710 (e.g., ribs 1719) makes an interference fit with the surrounding bone to create an initial binding of the anchor to the bone. At the same time, in at least some variations of the suture anchor, a solid distal ring located at the distal end of the suture anchor 1710 provides structural integrity to keep the anchor intact while it penetrates into the bone.

Turning now to FIGS. 23A-C, when suture anchor 1710 has been advanced an appropriate distance into the glenoid, the free end 1738 of suture 1730 is pulled proximally while the distal end of inserter is held in position, thereby causing enlarged first end 1731 of suture 1730 to move proximally relative to a body of suture anchor 1710, forcing a distal portion of suture anchor 1710 to split and expand, in the manner shown in FIGs. 23B-23C, for example, to further bind suture anchor 1710 to the bone of the glenoid. In an example of the method, expansion of suture anchor 1710 occurs along some or all of a circumference of a generally cylindrical body of the suture anchor 1710, and there may be variations in the amount of expansion about the circumference of the generally cylindrical body, e.g., there may be greater expansion in a direction perpendicular to the direction of longitudinally-extending slits 1717. It should be appreciated that the location and magnitude of expansion of the generally cylindrical body can be controlled by the number and location of longitudinally-extending slits, the configuration of enlarged first end 1731 of suture 1730 and the configuration of the generally cylindrical body of the suture anchor 1710 (e.g., its channel 1714 and the associated side wall 1718 adjacent the channel), etc. The method may be performed using many variations of suture anchor 1710. In one specific example, pulling suture 1730 causes a deployment cylinder 1720 disposed within channel 1714 of suture anchor 1710 to slide upward with enlarged first end 1731 until deployment cylinder 1720 presses against step 1715 within channel 1714, as shown in FIG. 23C. In this arrangement, it is a radial outer surface of deployment cylinder 1720 that presses outward against an inner surface of wall 1718 of suture anchor 1710 to cause radial expansion. In another example, not shown, expansion of a generally cylindrical body of the suture anchor 1710 occurs at a zone where diameter of the channel changes significantly, with expansion occurring as an enlarged first end of the suture moves out of the comparatively larger diameter distal end portion of the channel and into the comparatively smaller diameter intermediate portion of the channel.

Once suture anchor 1710 has been initially bound and set in the glenoid, the guide is removed from the surgical site, leaving the suture anchor 1710 deployed in the glenoid and suture 1730 extending out through the labrum. Additionally, ends 1762, 1768 of shuttle 1760 also extend outward away from suture anchor 1710 at this stage of the method.

The process of deployment and anchorage may then be repeated as desired so as to deploy additional anchors through the labrum and into the glenoid, with each anchor having a suture end extending out through the labrum.

To secure the labrum to the suture anchor 1710 for one or more securement devices 1705, the following steps are performed for each securement device 1705. With suture 1730 passed through the labrum 1703 such that enlarged first end 1731 of suture 1730 is pulled sufficiently to bring deployment cylinder 1720 into abutment with step 1715, free end 1738 of suture 1730 is attached to suture engagement structure 1762 of shuttle 1760. Then, free end 1768 of shuttle 1760 is pulled as shown in FIG. 23B to secure the labrum 1703 to the deployed suture anchor 1710. During this step, shuttle 1760 may be fully passed through portion 1734 of suture 1730 so that a finger trap formed through portion 1734 is based solely on parts of suture 1730. The anchorage of labrum 1703 to suture anchor 1710 may be completed by cutting suture 1730 outside of the finger trap formed in portion 1734 of suture 1730, as shown in FIG. 23C. Upon formation of the finger trap through portion 1734 of suture 1730, portion 1734 is located external to anchor 1710.

In view of the modest holding power required to secure the labrum in place, suture anchor 1710 can have a very small size, much smaller than a typical bone anchor of the sort used to hold a ligament in place. By way of example but not limitation, suture anchor 1710 may have a length of 8.25 mm (0.325 inches), an outer diameter (unexpanded) of 1.60 mm (0.063 inches), and an outer diameter (expanded) of 2.03 mm (0.080 inches). This small size enables a minimal puncture to be made in the labrum (and hence a minimal hole to be made in the labrum), thus reducing potential damage to the labral tissue and enabling a more accurate puncture location through the labrum. The small size of suture anchor 1710 also allows the anchor to be placed closer to, or directly into, the rim of the glenoid or acetabular cup, without fear of the anchor penetrating into the articulating surfaces of the joint. This significantly reduces, or entirely eliminates, labrum eversion problems. Furthermore, the small size of the anchor significantly reduces trauma to the tissue of the patient.

In a variation of the anchorage step of the above example, the securement device 1705 may have a starting condition, i.e., undeployed arrangement where portion 1734 of suture is within channel 1714. In this variation, after free end 1738 of suture 1730 is attached to suture engagement structure 1762 of shuttle 1760 and free end 1768 of shuttle 1760 is pulled to anchor the soft tissue to suture anchor 1710, portion 1734 rises out of channel 1714 so that it is outside of suture anchor 1710 adjacent to trailing end 1711.

In another example, the method may involve the use of securement device 1705 along with a drill or awl and an inserter. In this example, the method may be performed in a similar manner to that described above except without the use of a guide. The drill may be utilized to create an opening in the bone, and the inserter may be used to deploy the securement device 1705 into the opening. The method may otherwise be performed as described. In still further examples, the method may be performed with an inserter without a drill or a guide.

In other embodiments, a method of tissue repair may involve the performance of method steps as described in commonly owned U.S. Pat. App. No. 17/825,569 ("the `569 Application"), assigned to Stryker Corporation, the entire disclosure of which is incorporated by reference herein, but utilizing one or more securement devices as contemplated by the present disclosure. Non-limiting examples of these methods include a rotator cuff repair utilizing two or more anchors, as shown in FIGs. 4-14 of the '569 Application and described in the accompanying specification. Such methods may be employed with suture anchors that respectively include sutures with two one-way tensioning mechanisms so that each implanted anchor has two free working suture ends. In the performance of these methods, free ends of the working sutures may be used to pass through soft tissue and connect back to the same or another suture anchor, as described in the '569 Application. Further variations, including the use of a single suture for two or more anchors, are described in greater detail in the '569 Application. In some of these methods, such as those described for FIGs. 4-9 of the '569 Application, the anchors may be placed underneath the soft tissue. In other examples, such as those described for FIGs. 10-14 of the `569 Application, the anchors may be placed laterally adjacent to the tissue. In still further examples, some anchors may be underneath and others may be laterally adjacent to the tissue.

In other examples, a method of anchoring soft tissue may involve the use of any combination of the securement devices shown in FIGs. 1A, 2A-C and 3-14B and 20A-23. Thus, for example, a repair may include two or more types of devices, such as two or more of securement devices 105, 105', 105", 205, 305, 405, 505, 605, 705, 805, 905, 1005, 1105, 1205, 1305, 1305' ,1405, 1405', 1505, 1505', 1605, 1605', 1705. And, in these examples, any one device may be included in a quantity of two or more.

### EMBODIMENTS

The disclosure will now further describe the following numbered embodiments. It will be appreciated that some or all of the embodiments summarize aspects of the disclosure as provided in the detailed description and the figures. Accordingly, the embodiments are also to be read in connection with the preceding paragraphs and the appended figures, and do not limit the disclosure. The features and preferences as described hereinabove apply also to the following embodiments.
Embodiment 1: A device for securing soft tissue to bone, comprising:
   a suture anchor capable of being positioned within a bone hole, the suture anchor including a first end, a second end, an outer wall extending along a length between the first end and the second end, and a hollow interior extending along at least a portion of the length;
   a first suture including a first end portion, a second end portion, and a hollow braid, the first suture passing through the outer wall of the suture anchor at least once, wherein at least a portion of the first suture is positioned within at least a portion of the hollow interior of the suture anchor;
   a shuttle including a length and a suture-engaging structure, the shuttle capable of being positioned within at least a portion of the hollow braid of the first suture;
   a second suture including a first end portion and a second end portion, one of the first or second end portions of the second suture coupled to the first suture, the other of the first or second end portions of the second suture capable of being coupled to the suture engaging structure, wherein the shuttle is capable of being actuated to move the other of the first or second end portions of the second suture into the hollow braid of the first suture.
Embodiment 2: The device of embodiment 1, wherein when the shuttle is positioned within at least the portion of the hollow braid of the first suture, the portion of the hollow braid of the first suture is positioned outside of the hollow interior of the suture anchor.
Embodiment 3: The device of embodiment 1 or 2, wherein the first suture is a cylindrical length of suture and the second suture is a flat length of suture.
Embodiment 4: The device of embodiment 1, 2 or 3, wherein the one of the first or second end portions of the second suture is coupled to one of the first or second end portions of the first suture by a chain-link structure.
Embodiment 5: The device of any of embodiments 1-4, wherein the one of the first or second end portions of the second suture is coupled to one of the first or second end portions of the first suture by an opposing end-to-end tuck splice.
Embodiment 6: The device of any of embodiments 1-5, wherein the one of the first or second end portions of the second suture is coupled to one of the first or second end portions of the first suture by opposing Chinese finger trap structures.
Embodiment 7: The device of any of embodiments 1-6, wherein at least two portions of the first suture are positioned outside of the hollow interior of the suture anchor, and the shuttle is positioned within the at least two portions of the first suture positioned outside of the hollow interior of the suture anchor, wherein the shuttle is capable of being actuated to move the other of the first or second end portions of the second suture into the hollow braid of the at least two portions of the first suture positioned outside of the hollow interior of the suture anchor. Embodiment 8: The device of any of embodiments 1-7, wherein none of the second suture is positioned within the hollow interior of the suture anchor.
Embodiment 9: The device of any of embodiments 1-8, wherein the one of the first or second end portions of the second suture is coupled to the first suture without passing through the hollow braid of the first suture.
Embodiment 10: The device of any of embodiments 1-9, wherein the first suture includes one or more splices through itself.
Embodiment 11: A device for securing soft tissue to bone, comprising:
   a suture anchor capable of being positioned within a bone hole, the suture anchor including a first end, a second end, an outer wall extending along a length between the first end and the second end, and a hollow interior extending along at least a portion of the length;
   a first suture including a first end portion, a second end portion, and a hollow braid, the first suture passing through the outer wall of the suture anchor at least once, wherein a portion of the first suture is positioned within at least a portion of the hollow interior of the suture anchor and another portion of the first suture encircles the suture anchor circumferentially around the outer wall at least one half of a complete rotation;
   a shuttle including a length and a suture-engaging structure, the shuttle capable of being positioned within the hollow braid of the portion of the first suture encircling the suture anchor;
   a second suture including a first end portion and a second end portion, one of the first or second end portions of the second suture coupled to one of the first or second end portions of the first suture, the other of the first or second end portions of the second suture capable of being coupled to the suture-engaging structure, wherein the shuttle is capable of being actuated to move the other of the first or second end portions of the second suture into the hollow braid of the first suture.
Embodiment 12: The device of embodiment 11, wherein the first suture further comprises a third end portion, the second end portion of the first suture coupled to one of the first or second end portions of the second suture and the first suture includes a first portion extending to the first end portion and a second portion extending to the third end portion.
Embodiment 13: The device of embodiment 12, wherein the first portion of the first suture is the portion of the first suture encircling the suture anchor circumferentially around the outer wall at least one half of a complete rotation.
Embodiment 14: The device of embodiment 12 or 13, wherein the second portion of the first suture passes through the outer wall of the suture anchor at least twice.
Embodiment 15: The device of embodiment 12, 13 or 14, wherein the first end portion and the third end portion of the first suture are coupled together and are positioned within the hollow interior of the suture anchor, through one of the first end or second end of the suture anchor from within the hollow interior to extending beyond one of the first end or second end of the suture anchor.
Embodiment 16: The device of any of embodiments 11-15, wherein the second end portion of the first suture includes a loop and the first portion and second portion of the first suture each extend from the loop.
Embodiment 17: The device of embodiment 16, wherein one of the first or second end portions of the second suture includes a loop, the loop of the first suture coupled to the loop of the second suture to form a chain-link.
Embodiment 18: The device of any of embodiments 11-17, wherein the first portion of the first suture passes in between the second portion of the first suture and the outer wall of the suture anchor at least once.
Embodiment 19: The device of any of embodiments 11-17, wherein at least two portions of the second portion of the first suture are positioned outside of the hollow interior of the suture anchor, and a second shuttle including a length and a second suture-engaging structure is positioned within the at least two portions of the second portion of the first suture positioned outside of the hollow interior of the suture anchor, wherein the second shuttle is capable of being actuated to move the other of the first or second end portions of the second suture, capable of being coupled to the suture-engaging structure, into the hollow braid of the at least two portions of the second portion of the first suture positioned outside of the hollow interior of the suture anchor.
Embodiment 20: The device of any of embodiments 11-19, wherein the first suture is a substantially cylindrical length of suture and the second suture is a substantially flat length of suture.
Embodiment 21: The device of any of embodiments 11-20, wherein the one of the first or second end portions of the second suture is coupled to one of the first or second end portions of the first suture by an opposing end-to-end tuck splice.
Embodiment 22: The device of any of embodiments 11-21, wherein the one of the first or second end portions of the second suture is coupled to one of the first or second end portions of the first suture by opposing Chinese finger trap structures.
Embodiment 23: A method of securing soft tissue to bone, comprising:
   obtaining the device of any of embodiments 11-22;
   positioning the suture anchor in bone;
   passing the other of the first or second end portions of the second suture around or through the soft tissue to be secured;
   coupling the other of the first or second end portions of the second suture, passed around or through the soft tissue to be secured, with the suture-engaging structure;
   actuating the shuttle to move the other of the first or second end portions of the second suture into the hollow braid of the first suture; and
   tensioning the other of the first or second end portions of the second suture, positioned through the hollow braid of the first suture, to tension the soft tissue against the bone.
Embodiment 24: A device for securing soft tissue to bone comprising:
   a suture anchor having a lumen coaxial with a length of the suture anchor;
   a first suture portion having a length extending from a first end to a second end, the first suture portion passing into and out of the lumen over at least part of the length moving toward the second end from the first end;
   a second suture portion having a length extending from a first end to a second end, the second suture portion disposed relative to the suture anchor such that a position of the first suture portion keeps the second suture portion proximate the suture anchor; and
   a working suture having a first end operatively connected to at least one of the suture anchor, the first suture portion and the second suture portion.
Embodiment 25: The device of embodiment 24, wherein the first suture portion and the second suture portion are part of a single two-tailed suture.
Embodiment 26: A device securing soft tissue to bone comprising:
   a suture anchor capable of being positioned within a bone hole, the suture anchor including a first end, a second end, an outer wall extending along a length between the first end and the second end, and a hollow interior extending along at least a portion of the length;
   a first suture including a hollow braid, the first suture passing through the outer wall of the suture anchor at least once and the first suture including an internally disposed portion positioned within the hollow interior of the suture anchor; and
   a second suture having a body extending from a first end portion to a second end portion, at least part of the first end portion being coupled to the first suture and at least part of the body passing internally through the hollow braid of the first suture, the second suture being entirely external to the suture anchor.
Embodiment 27: The device of embodiment 26, wherein the first suture includes an exteriorly disposed portion that encircles the suture anchor circumferentially around the outer wall at least one half of a complete rotation, the at least part of the body of the second suture passing internally through the hollow braid of the first suture in the exteriorly disposed portion of the first suture.
Embodiment 28: The device of embodiment 26 or 27, wherein the first suture further comprises an exteriorly disposed portion and a snail portion on opposite sides of the internally disposed portion, the snail portion having a first sub-portion external to the outer wall and a second sub-portion internal to the outer wall.

Although the invention herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure as defined by the appended claims.

## Claims

1. A device for securing soft tissue to bone, comprising:
a suture anchor capable of being positioned within a bone hole, the suture anchor including a first end, a second end, an outer wall extending along a length between the first end and the second end, and a hollow interior extending along at least a portion of the length;
a first suture including a first end portion, a second end portion, and a hollow braid, the first suture passing through the outer wall of the suture anchor at least once, wherein at least a portion of the first suture is positioned within at least a portion of the hollow interior of the suture anchor;
a shuttle and a suture-engaging structure, the shuttle capable of being positioned within at least a portion of the hollow braid of the first suture;
a second suture including a first end portion and a second end portion, one of the first or second end portions of the second suture coupled to the first suture, the other of the first or second end portions of the second suture capable of being coupled to the suture engaging structure, wherein the shuttle is capable of being actuated to move the other of the first or second end portions of the second suture into the hollow braid of the first suture.

2. The device of claim 1, wherein when the shuttle is positioned within at least the portion of the hollow braid of the first suture, the portion of the hollow braid of the first suture is positioned outside of the hollow interior of the suture anchor.

3. The device of claim 1 or 2, wherein the first suture is a cylindrical length of suture and the second suture is a substantially flat length of suture.

4. The device of claim 1, 2 or 3, wherein the one of the first or second end portions of the second suture is coupled to one of the first or second end portions of the first suture by a chain-link structure.

5. The device of any of claims 1-4, wherein the one of the first or second end portions of the second suture is coupled to one of the first or second end portions of the first suture by an opposing end-to-end tuck splice.

6. The device of any of claims 1-5, wherein the one of the first or second end portions of the second suture is coupled to one of the first or second end portions of the first suture by opposing finger trap structures.

7. The device of any of claims 1-6, wherein at least two portions of the first suture are positioned outside of the hollow interior of the suture anchor, and the shuttle is positioned within the at least two portions of the first suture positioned outside of the hollow interior of the suture anchor, wherein the shuttle is capable of being actuated to move the other of the first or second end portions of the second suture into the hollow braid of the at least two portions of the first suture positioned outside of the hollow interior of the suture anchor.

8. The device of any of claims 1-7, wherein none of the second suture is positioned within the hollow interior of the suture anchor.

9. The device of any of claims 1-8, wherein the one of the first or second end portions of the second suture is coupled to the first suture without passing through the hollow braid of the first suture.

10. The device of any of claims 1-9 wherein:
another portion of the first suture encircles the suture anchor circumferentially around the outer wall at least one half of a complete rotation;
the shuttle is capable of being positioned within the hollow braid of the portion of the first suture encircling the suture anchor.

11. The device of any of claims 1-9, wherein the at least a portion of the first suture is a first portion and the first portion and a second portions extend from an end portion of the first suture, the first portion passing through the outer wall of the suture anchor at least twice and the second portion encircling the suture anchor circumferentially around the outer wall at least one half of a complete rotation.

12. The device of claim 11, wherein optionally the end portion of the first suture includes a loop partially disposed within the hollow interior of the suture anchor.

13. The device of claim 12, wherein optionally one of the first or second end portions of the second suture includes a loop, the loop of the first suture coupled to the loop of the second suture to form a chain-link.

14. The device of any of claims 11-13, wherein the second portion of the first suture passes in between the first portion of the first suture and the outer wall of the suture anchor at least once.

15. The device of any of claims 11-14, wherein at least two portions of the first portion of the first suture are positioned outside of the hollow interior of the suture anchor, and a second shuttle including a length and a second suture-engaging structure is positioned within the at least two portions of the first portion of the first suture positioned outside of the hollow interior of the suture anchor, wherein the second shuttle is capable of being actuated to move the other of the first or second end portions of the second suture, capable of being coupled to the suture-engaging structure, into the hollow braid of the at least two portions of the first portion of the first suture positioned outside of the hollow interior of the suture anchor.
